(19) Europäisches Patentamt
European Patent Office
Office européen des brevets

(11) **EP 3 999 161 B1**

(12) **EUROPEAN PATENT SPECIFICATION**

(45) Date of publication and mention
of the grant of the patent:
**31.12.2025 Bulletin 2026/01**

(21) Application number: **20758342.8**

(22) Date of filing: **07.07.2020**

(51) International Patent Classification (IPC):
***A61M 25/01*** *(2006.01)*

(52) Cooperative Patent Classification (CPC):
**A61M 25/0125; A61B 34/30; A61B 34/73;
A61M 25/0113; A61M 25/0122; A61M 25/0127;**
A61B 2034/302

(86) International application number:
**PCT/IB2020/056374**

(87) International publication number:
**WO 2021/009615 (21.01.2021 Gazette 2021/03)**

(54) **ULTRAFLEXIBLE FLOW DIRECTED DEVICE AND SYSTEM**

ULTRAFLEXIBLE STRÖMUNGSGESTEUERTE VORRICHTUNG UND SYSTEM

DISPOSITIF ET SYSTÈME D'ÉCOULEMENT DIRIGÉ ULTRASOUPLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.07.2019 EP 19186379**

(43) Date of publication of application:
**25.05.2022 Bulletin 2022/21**

(73) Proprietor: **ECOLE POLYTECHNIQUE FEDERALE
DE LAUSANNE
(EPFL)
1015 Lausanne (CH)**

(72) Inventors:
• **PANCALDI, Lucio
1025 ST-Sulpice (CH)**
• **SAKAR, Mahmut Selman
1028 Preverenges (CH)**
• **DIRIX, Pietro
8006 Zürich (CH)**

(74) Representative: **Grosfillier, Philippe et al
André Roland SA
IP Attorneys & Services
Avenue Collonges 21
1004 Lausanne (CH)**

(56) References cited:
WO-A1-00/07652      US-A- 5 730 733
US-A1- 2007 270 781    US-A1- 2013 060 137
US-B1- 6 524 303      US-B1- 6 929 624

## Description

## Technical Field

The present invention belongs to the fields of microtechnology and devices for research and medical purposes, such as for instance catheters and catheter guides. In particular, the invention features a flow conveyed, steerable device, system and method for e.g. surgical operations.

## Background Art

The Seldinger technique is a well-known method for percutaneous insertion of catheters into a patient's blood vessels. Typically, a large-bore hypodermic needle is used to access the patient's vein or artery. Flashback of venous or arterial blood through the needle indicates to the physician when the tip of the needle is in the lumen of the blood vessel. A catheter guide is then inserted through the needle into the lumen of the blood vessel and the needle is withdrawn. A catheter is then inserted coaxially over the catheter guide into the lumen of the blood vessel. At this time, the catheter guide may be withdrawn, leaving the catheter within the lumen of the blood vessel.

The construction of the catheter guide is critical to the successful completion of the Seldinger technique. The catheter guide must be flexible enough to exit the hypodermic needle and make the sometimes sharp turn into the vessel lumen without damaging or passing through the opposite wall of the vessel. The catheter guide must also be flexible enough to follow any sharp bends or tortuosity in the vessel without damaging the vessel wall. However, such a flexible catheter guide may not be rigid enough to guide a catheter through the tissue and any sharp bends, tortuosity or narrowing in the vessel without pulling back or kinking. This can be extremely problematic in cases where successful catheterization is critical to the survival of the patient.

Standard endovascular catheters or guidewires depend on external mechanical force in order to enter and advance within a blood vessel. The inserted guidewire must therefore be sufficiently rigid in order to withstand the pushing force and avoid buckling, but must also be sufficiently flexible to steer through the tight turns of the vascular system. These conditions impose fundamental limitations in miniaturization and therefore have limits in reachability, i.e. the ability of a catheter or guidewire to advance in an intricate vascular network. To address these limitations, flow-assisted infusion catheters were developed. In these devices, a flexible distal tip is assisted by the blood flow in order to reach tortuous locations (e.g. Magic catheter, Marathon catheter). Despite their flow-assisted distal tip, the catheters must be inserted mechanically and pushed towards the target location.

As an alternative technique, magnetic catheters with magnetic tips were developed. The performances of these catheters are remarkable inside cavities such as heart chambers in terms of stability, controllability and functionality. A variety of 3D magnetic manipulation systems is introduced to apply magnetic fields and magnetic field gradients in a controllable fashion. However, the navigation strategy depends on bending of the catheter, which limits their size (typically above 0.7 mm in diameter) and once again rely on the pushability of the rod. Publication US 6,524,303 discloses a variable stiffness steerable magnetic catheter having a proximal end, a distal end and a lumen therebetween. Publication US 2007/0270781 discloses a medical delivery system for delivering a medically useful payload through the vasculature to a site of interest in a patient's body.

## Summary of invention

In order to address and overcome at least some of the above-mentioned drawbacks of the prior art solutions, the present inventors developed a flow directed medical device with the features of claim 1, which might be embodied as a microcatheter, guidewire, sensor, endoscope and the like, having improved features and capabilities.

The purpose of the present invention is therefore that of providing a flow conveyed device that overcomes or at least reduces the above-summarized drawbacks affecting known solutions according to the prior art.

In particular, a first purpose of the present invention is that of providing a minimally invasive and miniaturized device that does not rely on pushing to be introduced and advanced along a tubular structure such as a blood vessel, but which is to the contrary entirely governed by a fluid flow, such as the blood flow.

A further purpose of the present invention is that of providing a flow directed device having the potential to reach any region in a subject's body at the speed of blood, thus reducing the duration of surgical operations and the related complications.

Still a further purpose of the present invention is that of providing a system and method to deflect and guide an extremely miniaturized and ultraflexible flow directed device such as the one described herein in an efficient and intuitive way, both for in vivo applications and in vitro/on chip applications.

All those aims have been accomplished with the present invention, as described herein and in the appended claims.

In view of the above-summarized drawbacks and/or problems affecting flow directed guidewires and catheters of the prior art, the present invention features a flow directed device according to claim 1.

Another object of the present invention relates to a system configured to release a flow directed device into the bloodstream according to claim 8.

Further embodiments of the present invention are defined by the appended claims.

The above and other objects, features and advantages of the herein presented subject matter will become more apparent from a study of the following description with reference to the attached figures showing some preferred aspects of said subject matter.

**Brief description of drawings**

In the Figures:

Figure 1 schematically depicts one embodiment of the device according to the present disclosure;

Figure 2 depicts the same embodiment of the device according to the present disclosure as depicted in Figure 1 further comprising a lumen spanning along the elongated body of the device and within it;

Figure 3 depicts the same embodiment of the device according to the present disclosure as depicted in Figure 1 further comprising conductive tracks and electrodes running along the elongated body of the device;

Figure 4 depicts the same embodiment of the device according to the present disclosure as depicted in Figure 1 further comprising comprising conductive tracks running along the elongated body of the device connecting distally-located sensors and a proximally-located external device such as a power supply or a computer-based analyser;

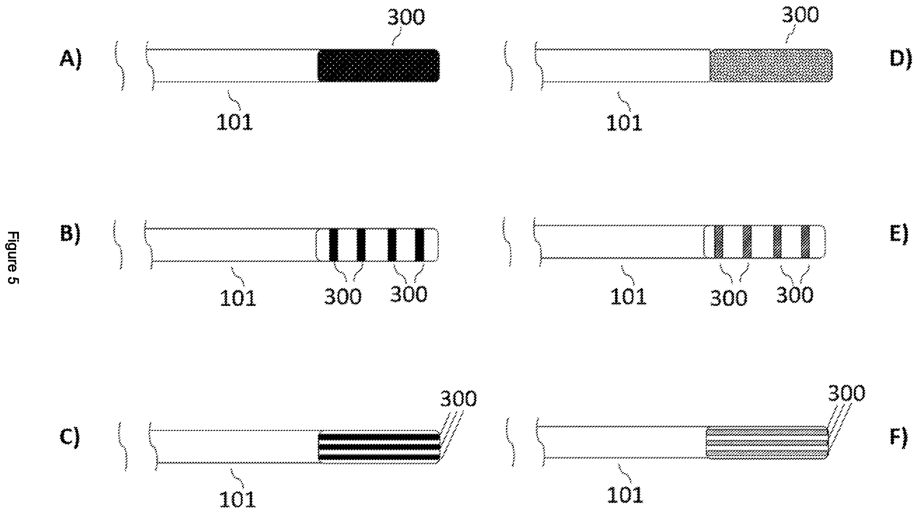

Figure 5 schematically depicts six embodiments of the ferromagnetic area or structure located on the distal end of the device: A) the distal end tip of the device consists of a metallic structure; B) the distal end tip of the device comprises a plurality of vertically-disposed metallic stripes; C) the distal end tip of the device comprises a plurality of horizontally-disposed metallic stripes; D)-E)-F): same embodiments as A), B) and C) wherein the metallic structures are substituted with a composite or polymeric material comprising a plurality of ferromagnetic particles or similar (flakes, nanotubes etc.);

Figure 6 schematically depicts the device of the invention A) at rest, when no magnetic field is applied and B) in a deflected state upon application of an external magnetic field;

Figure 7 schematically depicts two embodiments of the device of the invention comprising a drag member: A) one distally-located drag member; B) one distally-located drag member and a second drag member along the body of the device;

Figure 8 schematically depicts two further embodiments of the device of the invention comprising a drag member: A) one distally-located drag member and two drag members located along the body of the device; B) only two drag members located along the body of the device;

Figure 9 schematically depicts two further embodiments of the device of the invention comprising a drag member: A) one drag member located at the very tip of the device; B) one drag member located at the very tip of the device and two drag members located along the body of the device;

Figure 10 schematically depicts four configurations of the drag member: A) dome-like member; B) pyramid-like member; C) flat member; D) balloon;

Figure 11 schematically depicts the same six embodiments of the ferromagnetic area or structure located on the distal end of the device as described for Figure 5, wherein the ferromagnetic area or structure is comprised within the drag member;

Figure 12 schematically depicts one embodiment of the system according to the present invention;

Figure 13 schematically depicts three embodiments of the system of the invention having three different configurations for the insertion system: A) the releasing unit is embodied as a rigid member connected to the flow directed device; B) the releasing unit is embodied as a rigid member connected to a ball screw actuator; C) the releasing unit is embodied as a rigid member connected to a ball screw actuator, connected in turn to a soft, serpentine-like elongated member;

Figure 14 schematically depicts the same embodiment of the system according to invention as described for Figure 12 further comprising a stepper motor operatively connected to the releasing unit comprising a spool: A) lateral view; B) top view;

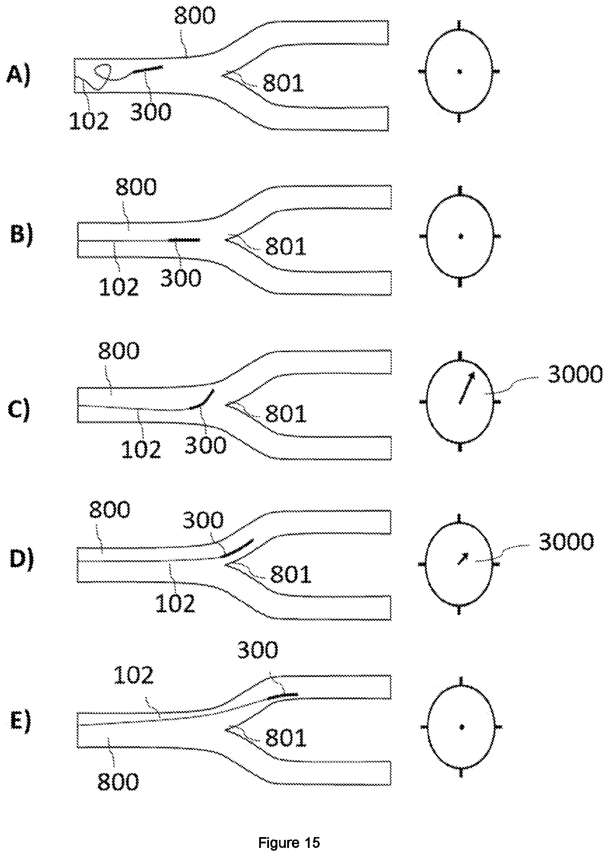

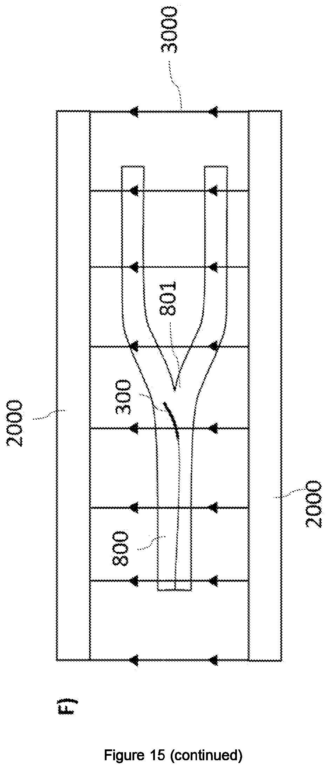

Figure 15 schematically depicts one embodiment of a method for guiding a flow directed device into a subject's body according to the invention: in a first step (A) it is shown a buckled device into a branched tubular structure such as a vasculature network. Upon release of a fluid such as aq physiological solution into the tubular structure (B), the device is tensioned and ready to advance. When approaching a branch or turn of the vasculature, the magnetic field is actuated (C) through electromagnets (as further shown in F) ), bringing to a deflection of the distal tip of the device. The device is therefore further advanced (D) thanks to the actuation of the releasing unit (e.g. a spool) into one branch, so that it can continue along the chosen path (E);

Figure 16 schematically depicts one embodiment of the system of the invention in which pumping means and the releasing unit are embodied as a single element fluidically connected with the insertion member. In this embodiment, the velocity of advancement of the plunger is equal to the velocity of the liquid inside the reservoir, said velocity being however much smaller compare to the velocity of the liquid inside the insertion member;

Figure 17 shows the flow-driven deployment and advancement of endovascular μprobes: the ultra-flexible μprobes are transported inside vessels by the hydrodynamic forces. Controlled rotation of the magnetic head using external magnetic fields steers the device into the target vessel. The small size of the

μprobe allows direct release into the vessels with standard hypodermic needles. Effective deployment of the μprobes via cannulation uses a system that overcomes mechanical instabilities. Perfusion with a physiological solution during delivery keeps the structure under tension until the flow in the artery takes over;

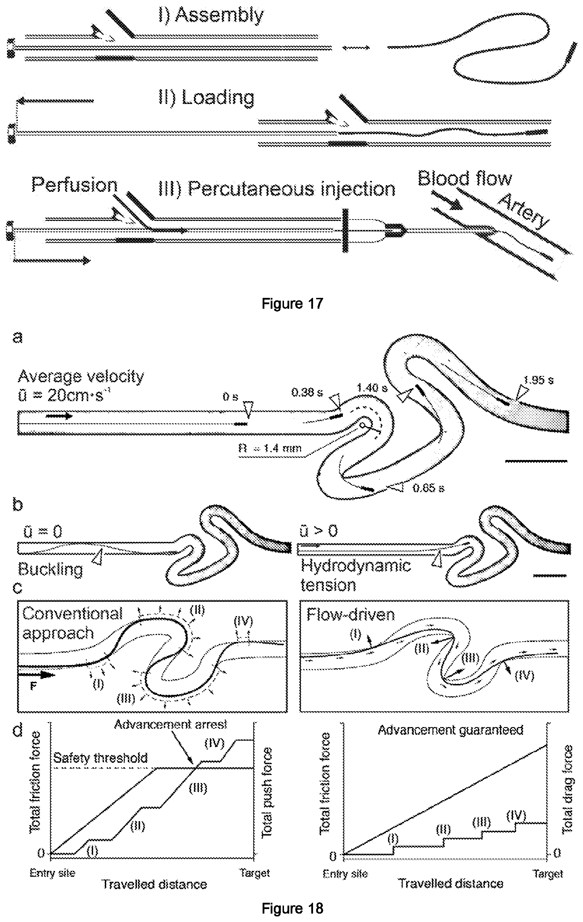

Figure 18 shows the characterization of the flow-driven autonomous advancement of μprobes. a Slender μprobes are transported by the flow inside tortuous channels with high curvature at a forward velocity of 2.8 cm·s⁻¹. The average flow velocity is given by $\overline{u}$ and R denotes the radius of curvature of the first turn. b In the absence of flow, pushing the μprobe forward generates mechanical instabilities and the advancement ceases (left). Upon introduction of the flow, the μprobe re-engages with it and continues to advance inside the channel. c A comparison between the conventional catheter advancement approach and fluid-driven transport introduced in this work. Conventional approach relies on a finite proximal force, F, and the contact pressure exerted on the outward wall to transmit forces distally (left). Our approach relies instead on the tensile forces applied by the viscous stresses along the entire structure (right). d In conventional navigation paradigm, friction forces increase with the length of the filament. The externally applied push force must be increased accordingly to be able to maintain the advacement. However, input forces above a safety threshold may result in vessel perforation or excoriation. Ultimately, when the friction exceeds the push force, the intervention reaches its limit. On the other hand, in our approach, the continuous presence of hydrodynamic forces maintains tension on the structure and warrants a positive difference between the drag force and the friction, regardless of the travelled distance. Plots are conceptual. Scale bars, 5 mm ;

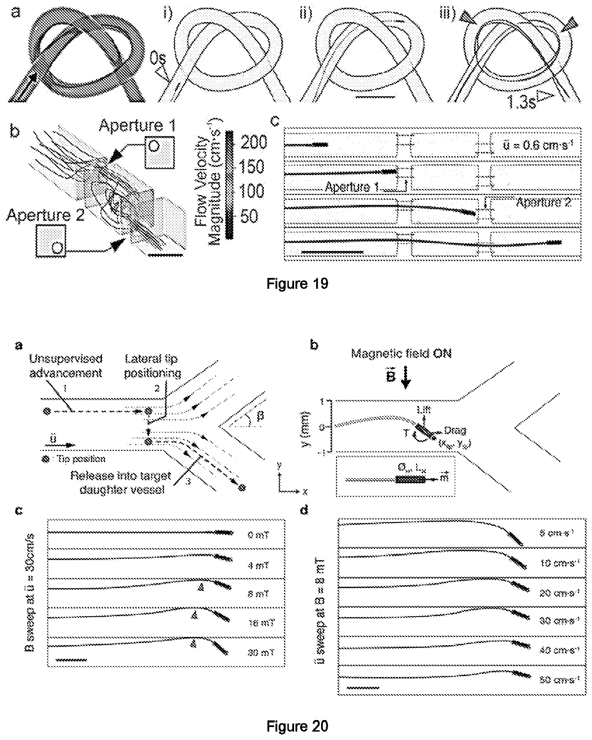

Figure 19 shows a simulation of the μprobe dynamics and adaptive navigation: a Schematic illustration of a 3D channel with the knot shape and time-lapse images of the μprobe (i-iii) advancing through a channel with the same geometry. The μprobe successfully reached the target location in 1.3 s with a forward velocity of 4 cm·s⁻¹. Grey arrows indicate points of contact with the wall. b CFD simulation of the flow in a channel with extreme occlusions. The main channel has 2 mm x 2 mm cross-sectional area and 750 μm-diameter holes were placed on two walls in the top-left and bottom-right corner, respectively. Scale bar, 2 mm; c Snapshots from the advancement of the μprobe inside the structured channel simulated in (b), finding its way through the holes even at very low flow velocity ($\overline{u}$ = 0.6 cm·s⁻¹). Scale bars in a and c, 5 mm;

Figure 20 shows magnetic tip steering for controlling the μprobe position: a Conceptual description of our proposed strategy to navigate the μprobe into the target daughter vessel. Upon reaching a bifurcation, the μprobe tip is laterally moved from the current stream (blue dashed lines) into the target stream (brown dashed lines) with the application of magnetic torque. The μprobe is pulled into the stream with further release of the structure. Black dashed line shows the trajectory of the tip passing through the stagnation streamline (white dashed line) and $\beta$ denotes the bifurcation angle. b The transveral position of the tip, $y_{tip}$, is controlled by the magnetic actuation of the head that has length $L_H$, diameter $\phi_H$, and magnetisation m. Upon application of the magnetic field, B, the torque exerted on the head rotates the head and bends the filament leading to an increase in both lift and drag forces. Red dot indicates the tip position. c The equilibrium shape of the μprobe with increasing magnetic field at constant flow velocity ($\overline{u}$ = 30 cm·s⁻¹). Red dot indicates the tip position. d The equilibrium shape of the μprobe with increasing flow velocity at constant magnetic field (B= 8 mT). Red dot indicates the tip position;

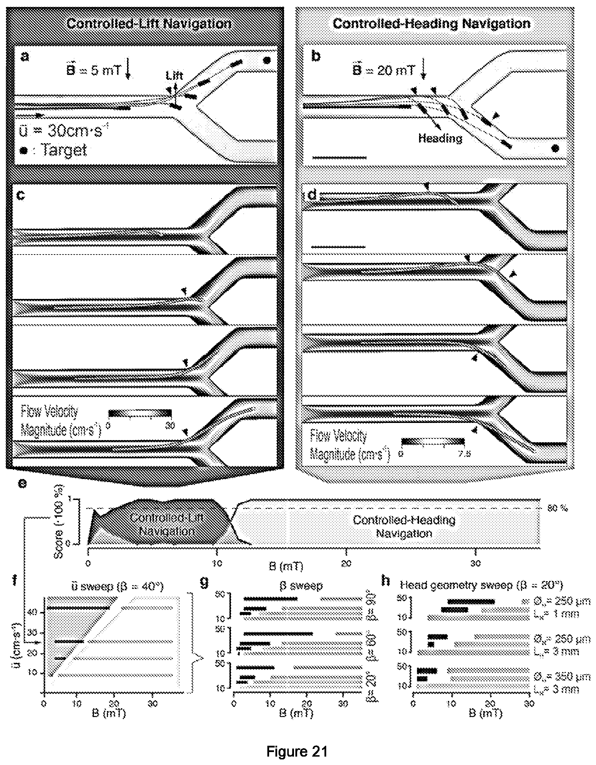

Figure 21 shows navigation strategies to deploy μprobes at target locations in the vasculature: **a,b** Overlay of time-lapse images showing controlled transport of μprobes to the target location (black dot) using **(a)** CL and **(b)** CH navigation. At lower *B*, CL navigation exploits the lift force emerging from the elastohydrodynamic coupling between the flow and the flexible structure. Lift force drifts the entire device into the upper daughter vessel (left). At higher B, the stronger magnetic torque enables setting of a course along which the μprobe is transported by the flow (right). **c,d** Simulations showing the successful navigation of the structure adopting **(c)** CL or **(d)** CH methods. **e** A score plot quantifying the statistics of the success rate for each navigation strategy at different magnetic field strength and constant flow velocity ($\overline{u}$ = 26 cm·s⁻¹). The μprobe was placed in the middle of a 2 mm x 2 mm channel bifurcating at an angle $\beta$ = 40° . Navigation tests were repeated 3 times for both daughter arteries with 3 different μprobes (n = 18). **f** Phase diagram illustrating the most favourable navigation strategy for given $\overline{u}$ and *B*. The values are binarized at an 80% confidence. **g** Effects of $\beta$ on the success of each navigation strategy. **h** The influence of the magnetic head geometry on the navigation score. Scale bars, 5 mm;

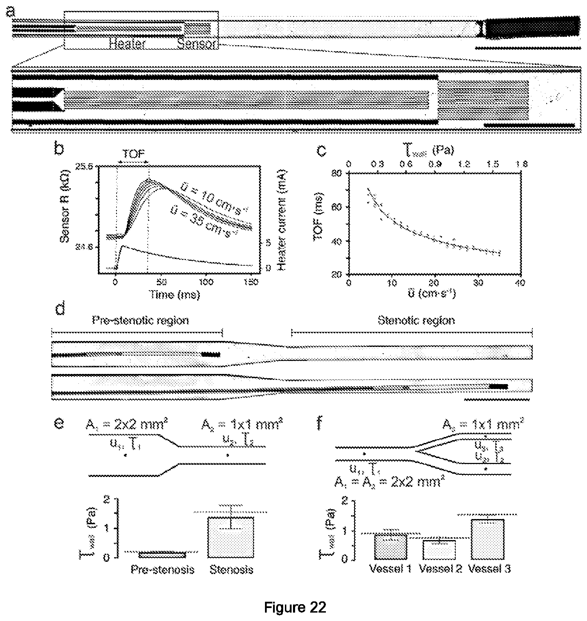

Figure 22 shows the development and navigation of microengineered thermal flow sensors. **a** Picture of the flow sensing μprobe showing the heater and sensor elements along with the magnetic head. Scale bar, 1 mm. Inset shows the details of the heater and the sensor circuits placed 50 μm apart from each other. Scale bar, 500 μm. **b** Temporal variation in the resistance of the sensor is recorded and compared with the input signal to measure the TOF of the heat pulse. Dark lines represent the average of 30 consecutive measurements that are shown in light curves. The TOF is measured for varying $\overline{u}$ in a 2 mm x 2 mm channel. **c** Calibration curve of TOF with respect to $\overline{u}$ and the $\tau_{wall}$ in a 2 mm x 2 mm channel. **d** Representative images showing the $\mu$probe at a pre-stenotic region and inside the stenosis. The cross-sectional area decreases 4 times inside the stenotic region. Scale bar, 5 mm **e** Wall shear stress measurements taken in the stenotic model shown in (d), after calibration with the curve shown in (c). The histograms are an average of 8 different trials with 30 measurements made at each trial. The dashed lines show the analytical calculations; error bars represent average $\pm$ standard deviation. **f** Wall shear stress measurements taken in a bifurcating channel at three different locations (black bullets), after calibration using the curve showin in (c). Histograms represent 10 different trials with 30 measurements at each trial ;

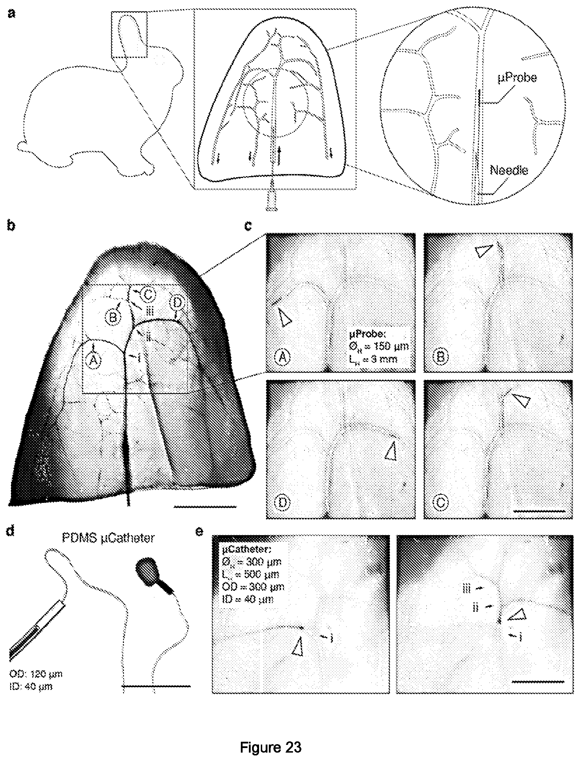

Figure 23 shows navigation and operation of $\mu$probes in perfused ex vivo rabbit ears: **a** Schematic representation of the vascular system in the rabbit ear. Red arrow marks the central artery ramification and blue arrows indicates the venous system. **b** Ink-perfused ear at the proximal central artery, highlighting the main arterial branches. Bifurcation are marked with roman letters (i, ii, and iii) while the targets locations are marked with capital letters (A, B, C and D). Scale bar, 20 mm. **c** The $\mu$probe with head dimensions $\phi_H = 150$ $\mu$m $L_H = 500$ $\mu$m was proximally inserted into the vasculature using the perfusion-based insertion system at a flow velocity between 0.5-1.5 cm·s$^{-1}$. White dashed lines outline the $\mu$probe for clarity. Scale bar, 10 mm. **d** A picture of the magnetic $\mu$catheter tube. A blue ink is injected through the $\mu$catheter. Scale bar, 5mm. **e** Positioning of the $\mu$catheter head at target locations and localized injection of the ink. Scale bar, 10 mm ;

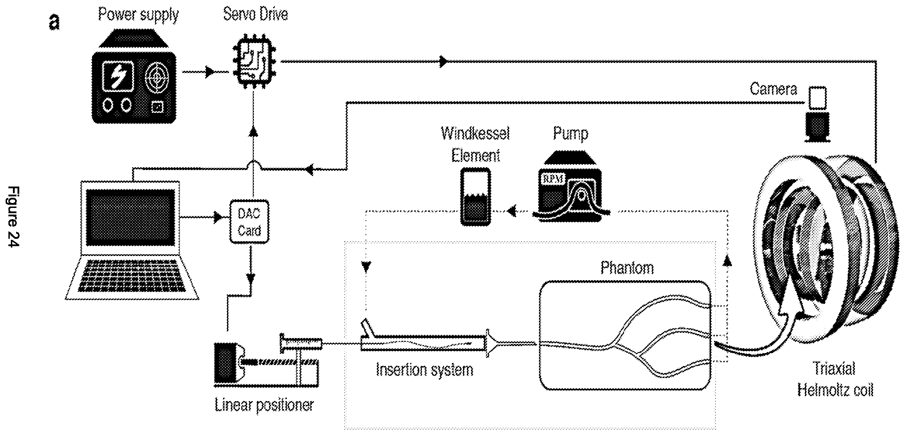

Figure 24 shows the experimental platform. Digital to Analog converter (DAC) card allows real-time communication between the computer and the hardware. The servo drives adjust the current provided by the power supplies to the coils. The linear positioner allows controllable release of the $\mu$probe through the rigid rod of the insertion system. Windkessel system is placed between the peristaltic pump and the perfusion-based insertion system to dampen the flow oscillations from the pump. Visual feedback for teleoperation is provided by a CMOS camera.

## Detailed description of the invention

The subject matter herein described will be clarified by means of the following description. It is however to be understood that the subject matter described in this specification is not limited to the aspects described herein and depicted in the drawings; to the contrary, the scope of the subject-matter herein described is defined by the claims. Moreover, it is to be understood that the specific conditions or parameters described and/or shown in the following are not limiting of the subject matter herein described, and that the terminology used herein is for the purpose of describing particular aspects by way of example only and is not intended to be limiting.

Unless otherwise defined, technical and scientific terms used herein have the same meaning as commonly understood by one of ordinary skill in the art to which this invention belongs. Further, unless otherwise required by the context, singular terms shall include pluralities and plural terms shall include the singular. The methods and techniques of the present disclosure are generally performed according to conventional methods well known in the art and as described in various general and more specific references that are cited and discussed throughout the present specification unless otherwise indicated. Further, for the sake of clarity, the use of the term "about" is herein intended to encompass a variation of +/-10% of a given value.

The following description will be better understood by means of the following definitions.

As used in the following and in the appended claims, the singular forms "a", "an" and "the" include plural referents unless the context clearly dictates otherwise. Also, the use of "or" means "and/or" unless stated otherwise. Similarly, "comprise", "comprises", "comprising", "include", "includes" and "including" are interchangeable and not intended to be limiting. It is to be further understood that where for the description of various embodiments use is made of the term "comprising", those skilled in the art will understand that in some specific instances, an embodiment can be alternatively described using language "consisting essentially of" or "consisting of."

In the frame of the present disclosure, the expression "operatively connected" and similar reflects a functional relationship between the several components of the device or a system among them, that is, the term means that the components are correlated in a way to perform a designated function. The "designated function" can change depending on the different components involved in the connection; for instance, the designated function of pumping means operatively connected with a liquid reservoir is that of applying a positive or negative pressure on said reservoir in order to create a liquid flow. A person skilled in the art would easily understand and figure out what are the designated functions of each and every component of the device or the system of the invention, as well as their correlations, on the basis of the present disclosure.

The term "subject" as used herein refers to animals and in particular mammals. For example, mammals contemplated by the present invention include humans, primates, domesticated animals such as cattle, sheep, pigs, horses, laboratory rodents and the like.

As used herein, a "ferromagnetic element" is any material or object that is attracted and/or deflected by the magnetic field produced by a magnet. A "magnet" is any material or object that produces a magnetic field. This definition includes permanent magnets, i.e. an object that creates its own persistent magnetic field, or even electromagnets, a type of magnet in which the magnetic field is produced by an electric current running through closely spaced turns of (usually metallic) wire. Both the magnets and the ferromagnetic materials can be made of elements well-known for their ferromagnetic properties such as iron, nickel, cobalt, rare earth metals such as neodymium or samarium, various kind of alloys or composite thereof such as alnico, ferrite or samarium-cobalt, oxides thereof, naturally-occurring minerals such as lodestone, and many others, as well as any combination of the aforementioned.

As used herein, "treatment", "treating" and the like generally mean obtaining a desired pharmacological and/or physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" or "treating" as used herein covers any treatment of a disease in a mammal, particularly a human, and includes: (a) inhibiting the disease, i.e., arresting its development; or (b) relieving the disease, i.e., causing regression of the disease and/or its symptoms or conditions such as improvement or remediation of damage. As used herein, the term "prevention" or "preventing" relates to hampering, blocking or avoid a disease from occurring in a subject which may be, for any reason, predisposed to the disease but has not yet been diagnosed as having it for example based on familial history, health status or age.

The term "flexible" is used herein to refer to all or part of the device of the invention that can undergo a deflection, either passively (i.e. without the presence of a deflecting force acting on the device except gravity) or actively (i.e. upon application of a bending force). Generally speaking, the term "deflection" refers to any displacement, expansion, contraction, bending, torsion, twist, linear or area strain, or any other kind of deformation, of at least a portion of the device of the invention. Preferably, a deflection is a bending.

The expression "flexural rigidity" refers to the force couple required to bend a non-rigid structure in one unit of curvature or it can be defined as the resistance offered by a structure while undergoing bending. In a beam or rod, flexural rigidity (defined as *EI*) follows the local deflection equation for beams in curvilinear coordinates, also known as the equation of Euler-Bernoulli:

$$1/\rho = M/EI$$

where the curvature is $1/\rho = d\theta/ds$. The relationship between bending moment and beam deformation therefore reads as

$$EI\frac{d\theta}{ds} = M(s)$$

Which in Cartesian coordinates for large deflections and an initially horizontal beam can be written as

$$\frac{d\theta}{ds} = \frac{\dfrac{d^2y}{dx^2}}{\left[1 + \left(\dfrac{dy}{dx}\right)^2\right]^{3/2}} = \frac{M(x)}{EI}$$

where E is the Young's modulus (in Pa), *I* is the second moment of area (in $m^4$), *y* is the transverse displacement of the beam at *x*, and *M(x)* is the force couple at x. Flexural rigidity has SI units of $Pa \cdot m^4$ (which also equals $N \cdot m^2$).

As used herein, the word "sensing" relates to the ability of a sensor to detect the presence of an item, such as a compound, present in a sample through at least one type of analysis. Additionally, "sensing" might refer to the ability of a sensor to detect a physiological and/or physiopathological activity or parameter, such as an electrical activity, in e.g. a subject's tissue and/or organ.

The word "measuring" relates to the ability of a sensor to estimate one or more of the properties of an item or an environment, such as a compound, present in a sample, such as its concentration or its nature, through at least one type of analysis. Furthermore, in the frame of the present disclosure, the word "monitoring" relates to the ability of a sensor to control and/or record the trend of one or more variable properties of an item or an environment, such as a compound, present in a sample, such as its concentration over time, through at least one type of analysis. Additionally, "measuring" might refer to the ability of a sensor to estimate one or more of the properties of e.g. a subject's tissue and/or organ, such as the ability of a sensor to control and/or record the trend of one or more physiological and/or physiopathological activity or parameter, such as an electrical activity, in a subject tissue and/or organ, even over time.

The expression "conductive track" refers to any film, path, stripe, strand, wire or the like which is electrically conductive in nature. For the sake of clarity, the word "electrode" is herein used to mean the distal part of a conductive track which is in direct contact with e.g. a subject's tissue, fluid or otherwise sample, and might be interchangeable with the word "pad". However, in embodiments of the invention, the term "electrode" is used to mean both a conductive track and its distal, terminal portion (or pad) configured to interface with e.g. a biological tissue, fluid or otherwise sample. Conductive tracks according to the present disclosure are used to connect and/or close an electrical circuit, and are thus usually electrical connectors or "interconnects". A conductive track is generally a metallic element that conducts an electric current toward or away from an electric circuit, but can be made of any suitable electrically conductive material, including but not limited to metals such as Au, Pt, Al, Cu, Ir and the like, as well as any alloy, oxides and/or combinations thereof; conductive polymeric materials; composite material such as polymeric materials embedding metal particles and/or metal strands or stripes, including insulating materials functionalized with electrically conductive flakes or fibers, for example carbon-filled polymers; liquid metals, including alloys or oxides thereof, such as gallium or gallium-indium; electrically conductive inks; as well as any suitable combination thereof. Micro-lithography and/or micro-integrated electronics, among other techniques readily available in the art, can be adopted to fabricate the components of the electrodes.

The term "compliant" is intended to include any conformable structure which is compressible, reversibly compressible, elastic, flexible, stretchable or any combination thereof. A "compliant electrode" is any structure or element able to deliver an electric current, and adapted to change its shape according to the shape change of the support it adheres to, without substantially compromising mechanical and/or electrical performances. Examples of compliant electrodes known in the art include metal thin-films (including patterned electrodes, out-of-plane buckled electrodes, and corrugated membranes), metal-polymer nano-composites, carbon powder, carbon grease, conductive rubbers or conductive paints, a review of which is provided in Rosset and Shea (Applied Physics A, February 2013, Volume 110, Issue 2, 281-307), incorporated herein in its entirety by reference. As it will be apparent to those skilled in the art, built-in multilayers or stacks of several layers of any of the above polymeric, composite, metallic and/or oxide materials, as well as combinations thereof, are encompassed in the definition of compliant interconnect.

The expressions "film" or "thin film" relate to the thin form factor of all or part of the device of the invention, such as a support substrate and/or a conductive track. Generally speaking, a "film" or "thin film" as used herein relates to a layer of a material having a thickness much smaller than the other dimensions, e.g. at least one fifth compared to the other dimensions. Typically, a film is a solid layer having an upper surface and a bottom surface, with any suitable shape, and a thickness generally in the order of micrometers or nanometers, depending on the needs and circumstances, e.g. the manufacturing steps used to produce it. In some embodiments, films according to the invention have a thickness comprised between 0.1 $\mu$m and 500 $\mu$m, such as between 1 $\mu$m and 500 $\mu$m, between 5 $\mu$m and 500 $\mu$m, between 10 $\mu$m and 500 $\mu$m, between 5 $\mu$m and 300 $\mu$m, between 50 $\mu$m and 300 $\mu$m between, between 50 $\mu$m and 200 $\mu$m, between 100 $\mu$m and 500 $\mu$m, between 200 $\mu$m and 500 $\mu$m, between 5 $\mu$m and 50 $\mu$m or between 1 $\mu$m and 10 $\mu$m. Thin films according to the present disclosure are sized and shaped according to the above-listed thicknesses to comply to the inventive concept behind the invention, namely permitting a fluid flow-driven drag of the device along tubular structures, such as small and tortuous blood vessel networks, without performing any push-pull operation of the device.

A "tubular structure" is any structure having a hollow elongated body. The definition includes simple tubular structures or branched ones, as well as both straight or tortuous tubular configurations. Additionally, a tubular structure might have any shape in terms of contours and/or cross-sections, which may possibly vary along one or more portions thereof, and a cross-sectional size normal to the longitudinal axis spanning from micrometers to centimeters. A tubular structure might include for example microfluidic channels included into microfluidic chips, or bodily tubular structures including, but not limited to, gastrointestinal system structures including oesophagus, urinary system structures including urethra and ureters, blood vessels such as arteries and veins, the lymphatic system, biliary ducts, lacrimal ducts, respiratory system structures including trachea, bronchi and bronchioles, as well as phantom reproduction thereof.

In the frame of the present disclosure, a "soft" material is any material that is either compressible, reversibly compressible, flexible, elastic, stretchable or any combination thereof. The term "stretchable" is herein used to mean an intrinsic or engineered property of a material or structure that allows such material or structure to perform a large elongation upon a strain stress, typically of >5% of the elongation of a soft structure at rest, such as for instance more than about 10%, more than about 20%, more than about 50%, more than about 100% or even more than about 200% of a soft structure at rest, in an elastic behaviour regime and without or with minimal impairment of the mechanical and/or functional performances of the said material or structure along a single or multiple strain stress cycles.

The expression "tensile strength" refers to the measurement of the force required to pull a material or structure to the point where it breaks. The tensile strength of a material is therefore the maximum amount of tensile stress that a material or structure can withstand while being stretched or pulled before failure, for example breaking. There are three typical definitions of tensile strength: 1) yield strength, the stress a material can withstand without permanent deformation, which will cause a permanent deformation of 0.2% of the original dimension; 2) ultimate strength, the maximum stress a material can withstand; and 3) breaking strength, the stress coordinate on the stress-strain curve at the point of rupture.

The present invention features a novel navigation approach that does not require pushing a device, such as guidewires or catheters, to advance inside a tubular structure such as a blood vessel. Thanks to the inventive concept upon which the invention is based, the flow directed device herein described allows to achieve unprecedented results in an extremely efficient fashion. The device can navigate micrometer-sized arteries under the only influence of a fluid flow, e.g. the viscous blood flow, without the guidance of any pushable tool such as a guidewire, and can be externally operated at vessels bifurcations using a magnetic field, such as a spatially uniform magnetic field, by controlling the orientation of the distal magnetic tip. Thanks to the top level expertise of the inventors in the field of micro/nanofabrication, the device of the invention is engineered to be extremely flexible (cross-sectional area ranging from 20 to 15'000 $\mu m^2$ and Young's modulus ranging from kPa to GPa) in order to conformably comply with a fluid flow and be passively transported by a viscous drag, without being bond to any particular geometry or material. The device can therefore be simply un-winded into (fed to or injected into) the fluid stream at a velocity chosen by the user.

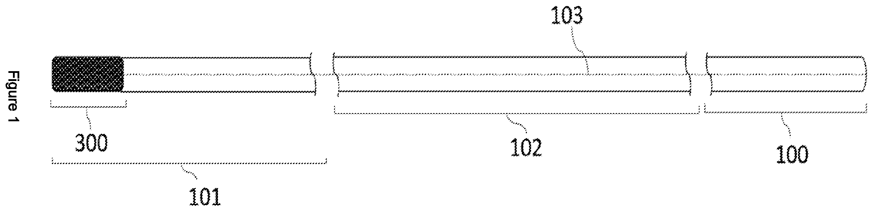

With reference to Figure 1, depicting one embodiment of the invention, a flow directed device **1** is herein disclosed adapted to be guided entirely and uniquely by a fluid flow flowing along a tubular structure, such as by the blood flow within a blood vessel, said device comprising a proximal end **100,** a distal end **101** and an elongated body **102** in between defining a longitudinal axis **103,** the device being characterized in that said distal end **101** comprises a ferromagnetic area or structure **300** capable of deflecting the distal end **101** when subjected to an external magnetic field.

A device according to the invention can be fabricated to have several different appearances and shapes such as for instance a solid tubular shape, a flat shape (a thin film), or a hollow tubular or flat shape, to mention At least the distal end **101** is configured as a ribbon. The shape and appearance are dependent of the suited function and application, without departing however from the inventive concept of the invention. As a way of example, a hollow device would be preferred in the frame of the present invention for all those applications needing delivery of a fluid to a target region in the body of a subject to be treated, thus rendering *de facto* the device a catheter or micro-/nanocannula; alternatively, nanodevices or optical fibers can be let flown or slid inside a tubular cavity obtained within the body of the device, thus allowing those tools to reach barely accessible spots in a subject's body through the vascular system. Further, and advantageously, embolization due to the operation of catheters and guidewires is avoided or at least limited with the device of the invention configured as a ribbon, as the blood would be allowed to flow as usual even in case of a blockage in an artery/vein.

While miniaturization of conventional guidewires or catheters is an open challenge due to the way they are navigated inside the arteries, the inventive concept according to the present invention exploits the small-scale features to mechanically adapt to the blood flow, eliminating the miniaturization issue by taking advantage of it. In other words, the present device can potentially be miniaturized further to be guided in the smallest arteriole.

Accordingly, the device of the invention can be fabricated with diameters (for tubular designs) ranging from about 500 nm to about 500 $\mu$m, such as between about 1 $\mu$m and about 300 $\mu$m, between about 10 $\mu$m and about 200 $\mu$m or between about 20 $\mu$m and about 100 $\mu$m such as about 50 $\mu$m. For instance, in one embodiment the device of the invention might have a hollow tubular shape with an inner diameter of about 40 $\mu$m and outer diameter comprised between about 100 and 200 $\mu$m. In some embodiments, the cross section (independent of the design or external surface shape) perpendicular to the longitudinal axis **103** has a dimension comprised between about 500 nm and about 100 $\mu$m such as between about 1 $\mu$m and about 50 $\mu$m, which is a range advantageous for applications envisaging e.g. a deep access into tiny and tortuous vasculature such as the brain vasculature. For the sake of clarity, a "cross section" is a linear dimension that might be selected from the thickness, the diameter, the side or the diagonal, depending on the geometry, of the device of the invention. These dimensions are considered to be the optimal choices in particular for biomedical applications, wherein tiny tubular structures such as brain capillaries are targeted as body tubular structures.

Alternatively, for flat devices, these can be implemented as a thin film element, having a flat appearance and a thickness comprised between about 500 nm and about 30 $\mu$m, such as between 1 and 10 $\mu$m, between 2 and 20 $\mu$m or between 1 and 5 $\mu$m, and a width comprised between about 10 $\mu$m to about 500 $\mu$m, such as between 30 and 300 $\mu$m, between 50 and 200 $\mu$m or between 50 and 100 $\mu$m. The device can come in various length, typically ranging from about 50 cm to about 350 cm or longer. As a way of comparison, typical guidewire for cardiovascular use in e.g. interventional radiology have diameters in the range of 0.014" to 0.034" (i.e. from about 350 to about 850 $\mu$m or more) and a length in the range of 100 to 400 cm.

In terms of materials, in preferred embodiments one or more of the portions composing the device of the invention (the entire device or at least the distal end **101** and the elongated body **102)** have an elastic modulus comprised between about 100 kPa and about 10 GPa, preferably between 500 kPa and 5 GPa such as between about 700 kPa and about 3.5 GPa, thus possibly facilitating, *inter alia,* the elastic compliance of the device with the pulsatile blood flow. In one embodiment the entire device or at least the distal end **101** and the elongated body **102** are substantially made of a soft polymeric material. Exemplary soft polymeric material can be selected from a non-limiting list comprising thermosets or thermoplastics such as styrene butadiene styrene (SBS) or styrene ethylene butylene styrene (SEBS), Nylon, Polyether block amide (PeBax) polyamide, Polyvinyl alcohol (PVA), polyimide (PI), poly ethylene (PE), poly propylene (PP), polyether etherketone (PEEK), Acrylonitrile butadiene styrene (ABS), epoxys, polytetrafluoroethylene (PTFE); soft foams such as polyurethanes (PU) including reticulated polyurethanes; flexible polyvinyl chloride (PVC), neoprene, uncrosslinked neoprene, cross-linked polyethylene (PE), polyethylene terephthalate (PET) polyether, ethylene-vinyl acetate (EVA), polyethylene-vinyl acetate (PEVA), polypropylene glycol (PPG), latex; elastomeric materials such as silicone rubber (e.g. polydimethylsiloxane PDMS) or fluorosilicone rubber; thermoplastic elastomers such as styrenic block copolymer (SBC), ethylene propylene diene monomer (EDPM) rubber, butyl rubber, nitrile rubber; natural polymeric materials (i.e., non-synthetic polymers, polymers that can be found in nature) and/or polymers derived from the Extra Cellular Matrix (ECM) as spider silk, gelatin, elastin, collagen, agar/agarose, chitosan, fibrin, proteoglycans, a polyamino-acid or its derivatives, preferably polylysin or gelatin methyl cellulose, carbomethyl cellulose, polysaccharides and their derivatives, preferably glycosaminoglycanes such as hyaluronic acid, chondroitinsulfate, dermatansulfate, heparansulfate, heparine, keratansulfate or alginate; anisotropic materials such as liquid crystal elastomers and architected composite hydrogels reinforced with collagen or cellulose fibers, or suitable combinations of any of the foregoing.

Alternatively, the entire device or at least the distal end **101** and the elongated body **102** can be substantially made of composite materials, super-elastic metals or various metals/alloys/oxides such as for instance Kevlar, stainless titanium, steel or stainless steel, nickel titanium alloy (Nitinol), nickel-chromium alloy, nickel-chromium-iron alloy, cobalt alloy, tungsten, cobalt, chrome, nickel, aluminum, copper, molybdenum or any combination thereof. These materials are the ones typically used in the context of medical devices such as guidewires or catheters, being easily sterilisable and manufactured, and biocompatible.

One important aspect addressed by the device of the invention is the distribution of the force along the device: for instance, the viscous drag force of the blood is mechanically propelling the device all along from its access point into the blood stream until its most distal point. This aspect can therefore promote safer passage than standard catheters or guidewires, which require a pushing force from their proximal end that is proportional to the distance travelled by the device, due to the increasing mechanical friction between the catheter/guidewire and the vessel walls. According to the above, therefore, independent of the material used, the flow directed device of the invention has a tensile strength, or ultimate strength, of at least its distal end **101** and/or its elongated body **102** comprised between about 5 and about 200 MPa, preferably between 50 and 100 MPa as measured by tensile testing, a range which is considered to be optimal in order to combine a sufficient resistance of the distal portion to drive the entire device along a blood vessel via the blood flow without failure such as breakage. As it will be evident for a person skilled in the art, the tensile strength of the device of the invention, in at least its distal end **101** and its elongated body **102,** is the result of a combination of design, materials and dimensions, which can be adapted on a case by case basis depending on the needs and circumstances.

Another key feature of the device according to the invention relates to its flexural rigidity, particularly in at least the distal end **101** and the elongated body **102.** Conventional guidewires or catheters mainly depend on external push; thus, the entire device needs to slide on the artery walls in order to be pushed forward. With increasing distance, the force applied on the arteries increase accordingly, which might have serious implications on the inner walls and raise the risk of puncture. In the device of the invention, the fluid flow itself limits the number of contact points with the artery walls, thus drastically reducing the risks of friction and excoriation. Moreover, because the forces are governed by the blood flow, the device cannot exert additional stress on the artery wall than the flow itself, eliminating any risk of puncturing the walls while providing autonomous haptic capabilities. Accordingly, in preferred embodiments of the invention, at least the distal end **101** and the elongated body **102** of the device have a flexural rigidity comprised between about $10^{-10}$ and about $10^{-14}$ $Pa \cdot m^{-4}$. As a way of comparison, typical guidewire for cardiovascular use in e.g. interventional radiology having a diameter of around 0.9 mm has usual flexural rigidities of around $10^{-3}$ $Pa \cdot m^4$, namely around ten orders of magnitude higher that the device of the invention. Again, the flexural rigidity is the result of a combination of design, materials and dimensions, which can be adapted on a case-by-case basis depending on the needs and circumstances, and which a skilled person would readily figure out and adapt without departing from the inventive concept of the invention.

In some embodiments, depending on the needs, the distal end **101** might be engineered or might comprise a material that will render it floppier (have a lower stiffness and a lower flexural rigidity) than the body **102** of the device, while in other circumstances the distal end **101** and the elongated body **102** may have substantially similar stiffness. The distal end **101** may extend typically from 0.5 to 20 cm, such as 1 to 5 centimeters.

With regards to the proximal end **100,** this might have different features in terms of mechanics, material and/or geometry. As will be clarified later on in the present disclosure, the proximal end **100** might comprise additional elements such as fluidic and/or electrical connections, might have a different, typically bigger, shape and/or size and might be composed of one or more materials with e.g. a higher Young's modulus, higher flexural rigidity and/or higher tensile strength in order to e.g. safely and reliably connect with an insertion system. Nonetheless, in some embodiments the proximal end **100** can have the exact or similar features than the distal end **101** and/or the body **102** of the device. The proximal end **100** can compose from about one tenth (1/10) to about one fifth (1/5) of the entire device, and may extend from about 5 to about 80 cm.

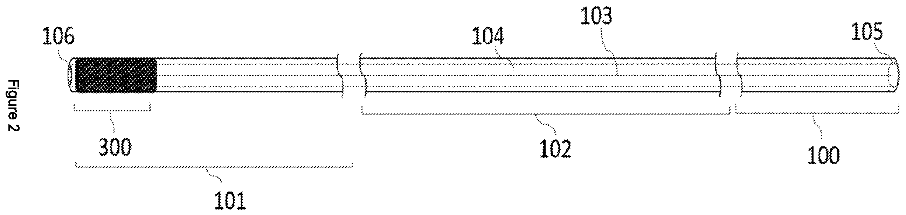

In embodiments of the invention, as the one depicted in Figure 2, the flow directed device comprises a lumen **104** spanning from the distal end **101** up to the proximal end **100** and along the entire body **102,** said lumen **104** being coaxially arranged compared to the longitudinal axis **103** of the device. The distal end portion **101** comprises or consists of a bendable portion, optionally having a tip at its very end generally being an atraumatic and non-sharp tip, with a rounded, oval, or similar appearance. Materials opaque to X-rays, such as platinum, gold, tungsten, barium sulfate, bismuth oxide, bismuth subcarbonate, iron, neodymium, boron, tantalum or the like, may be advantageously incorporated therein, to act as a fluoroscopic marker to aid in visualization.

Lumen **104** is preferably embodied as a channel, such as a microfluidic channel, having a proximal end inlet **105** and a distal end outlet **106** configured to transport for instance a fluid, rendering *de facto* the flow directed device of the invention a fluidic/microfluidic device such as a catheter, depending on the needs and circumstances. In some embodiments, the device comprises a plurality, such as an array, of channels or microchannels.

The fluidic channels may be used for instance to supply a compound, such as a pharmaceutical compound, a gas, a buffer medium and the like, to a target body site in order to e.g. pharmacologically stimulating a body tissue. The fluidic channels of the device according to the invention can be used e.g. for delivering drugs such as vasospasm relief drugs or others, to remove locally unfavourable chemical products or waste products, or even to deliver surgical adhesives for fixation purposes. For this, a connection of at least some of the fluidic channels to the outside can be provided via a proximal end inlet **105** of the flow directed device. Additionally or alternatively, the channels can be operatively (fluidically) connected through a proximal end inlet **105** to external devices such as a syringe, a fluidic pump (e.g. mechanical pumps or micropumps, peristaltic pumps and the like) or some other drug delivery device via any suitable connection means. Additionally or alternatively, the channel(s) can be used to transport elements such as nanodevices or optical fibers.

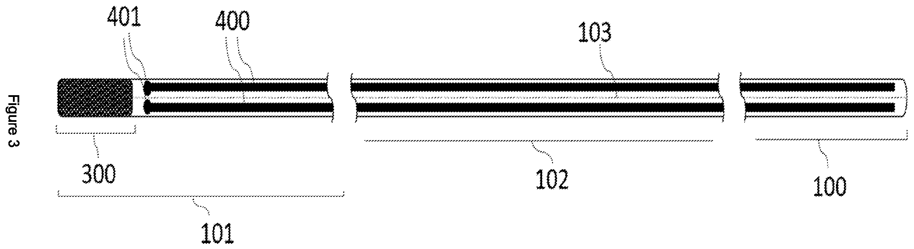

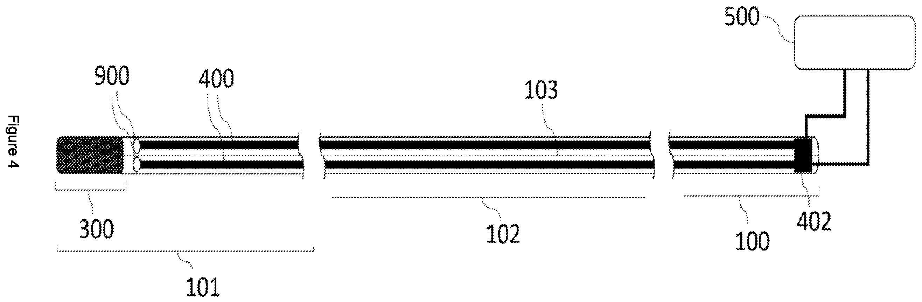

In additional or alternative embodiments according to the invention, the device comprises electrically conductive tracks **400** (Figure 3). Typically, in one set of embodiments, said conductive tracks **400** act as interconnects between a subject's tissue and further electrical and/or electronic devices such as for instance electrical stimulators, PCBs, microchips, connectors, wires and the like. Conductive tracks **400** can preferably comprise a distal, end electrode portion or pad **401** configured to directly interface a bodily tissue, such as an artery wall. Additionally or alternatively, conductive tracks **400** electrically connect two or more electrical or electronical devices between them, typically located at two different positions along the flow directed device: as a way of example, conductive tracks **400** can electrically join one or more proximally-located devices such as power supplies **500** with sensors **900** such as temperature sensors, velocity sensors, pressure sensors and the like distally located along the flow directed device (Figure 4).

Conductive tracks **400** and/or electrodes **401** can be made of any suitable electrical conductive material, including but not limited to metals such as Au, Pt, Al, Cu, Pt-Ir, Ir, and the like, as well as any alloy thereof, oxide thereof and combinations thereof, composite metal-polymer materials, such as Pt-PDMS composites or Pt-Ir-PDMS composites or Ir-PDMS composites and so forth, as well as conductive polymers such as poly(3,4-ethylenedioxythiophene) polystyrene sulfonate (PEDOT:PSS). In a preferred embodiment, the electrodes are made of non-toxic and biocompatible materials. Conductive tracks **400** and/or electrodes **401** can be placed on or within the substrate composing the flow directed device by any suitable means such as for instance photolithography, electron beam evaporation, thermal evaporation, sputter deposition, chemical vapour deposition (CVD), electro-plating, molecular beam epitaxy (MBE) or any other conventional means known in the art. Conductive tracks **400** might be further at least partially encapsulated to avoid short circuits and failure thereof, i.e. passivated whilst leaving the electrodes **401** exposed through connecting vias.

In a set of embodiments according to the invention, conductive tracks **400** are compliant interconnects. A "compliant interconnect" is any structure or element able to deliver an electric current, and adapted to change its shape according to the shape change of the support it adheres to, without substantially compromising mechanical and/or electrical performances. As it will be apparent to those skilled in the art, built-in multilayers or stacks of several layers of any of the above polymeric, composite, metallic and/or oxide materials, as well as combinations thereof, are encompassed in the definition of compliant interconnect. In some embodiments, stretchable interconnects as the ones described in International Patent Applications WO 2004/095536, WO 2016/110564 and/or WO 2018/100005A1 can be used.

In one set of embodiments, the biomedical device of the invention comprises one or more arrays of elastically stretchable microelectrodes. As used herein, for the sake of clarity and conciseness, a "stretchable microelectrode array" refers to the ensemble of a plurality of interconnects **400** and distal electrodes **401** having the ability to withstand mechanical deformations such as flexing, stretching, torsion or the like, without electrical failure or loss of their electrical features. Stretchable arrays (for instance stretchable gold and/or Cr/Au microelectrode arrays, MEAs) are becoming more and more popular and find convenient applications in various fields. It has in fact been verified that the impedance of microelectrode arrays stay low and stable during the deformation and even after repeated torsions, and therefore facilitate e.g. the recording of small amplitude biological signals and ensure efficient functional electrical stimulation.

The electrical connection between electrodes interconnects **400** and one or more electrical and/or electronic external device **500** can be done in any suitable way. With reference for instance to Figure 4, in one embodiment some connection wires run from the interconnects **400** to the one or more external electrical and/or electronic device **500,** where e.g. an electrical pad electrically couples said wires to device **500.** Connection means **402** for electrical and/or magnetic connection with external devices can be further present in the device of the invention, implemented for instance as electrical pad(s) for establishing an electrical connection between electrodes interconnects **400** and one or more electrical and/or electronic device **500** via wired connections; additionally or alternatively, connection means **402** can be implemented as wireless means for coupling the electrode(s) with e.g. external receivers in a wireless mode, for instance through (resonant) inductive coupling, (resonant) capacitive coupling, magnetodynamic coupling, ultrasounds and/or infrared radiation, by the simple implementation of solenoid antennas or coils. As it will be apparent to a person skilled in the art, connection means **402** are preferably located proximally in the flow directed device, more preferably in the proximal end **100** and most preferably in the very proximal end tip of the proximal end **100.**

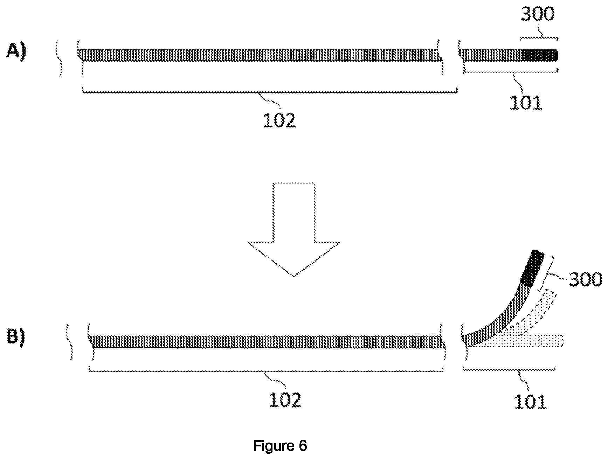

While the transportation of the device is passively governed by a fluid flow, the guidance thereof (i.e., steering) is actively performed wirelessly using an external magnetic field able to influence the deflection of the distal end portion **101**. In this context, as anticipated, the flow directed device of the invention comprises at least one magnetic area or structure **300,** preferably in or on the distal end portion **101,** which is configured to be magnetically influenced by an external magnetic field in a way to deflect said distal end portion **101** (Figures 5 and 6). The magnetic area or structure **300** can be located along the entire distal end portion **101** or only part of it, and can be coupled onto/into said distal end portion **101** by methods known in the art such as by soldering, adhesives, doping of ferromagnetic particles into the distal end portion **101** and the like. In embodiments of the invention, the magnetic area or structure **300** comprises at least one of ferromagnetic particles, ferromagnetic stripes, a ferromagnetic cylinder, ferromagnetic ribbons, ferromagnetic films and ferromagnetic filaments. In additional or alternative embodiments, the distal end portion **101** or only part of it can be in itself the magnetic area or structure **300,** i.e. can comprise or consist of ferromagnetic materials adapted to be influenced by an external magnetic field. In one embodiment, the distal end portion **101** or only part of it can be composed of a composite material manufactured by casting soft elastomeric polymers such as PDMS doped with hard ferromagnetic microparticles. The structure is subsequently magnetized with the application of a high magnetic field (e.g. >2.5 T). Typically, the ferromagnetic area or structure **300** is capable of deflecting the distal end **101** when subjected to an external magnetic field comprised between about 1 mT and 500 mT, such as between 1 mT and 100 mT. The magnetic field needed to deflect the distal end **101** is function of the size of the ferromagnetic area or structure **300**; as the torque created by a magnetic field on a 3D volume scales with the cube of the volume, for magnetic structures **300** of a volume comprised between about 0.02 $mm^3$ and about 3 $mm^3$ a magnetic field of 30 mT suffices to create enough displacement of the structure to be able to steer the tip of the flow directed device. For volumes below 0.02 $mm^3$ the magnetic field has to be significantly increased, whereas for volumes above 3 $mm^3$ much less magnetic field is necessary.

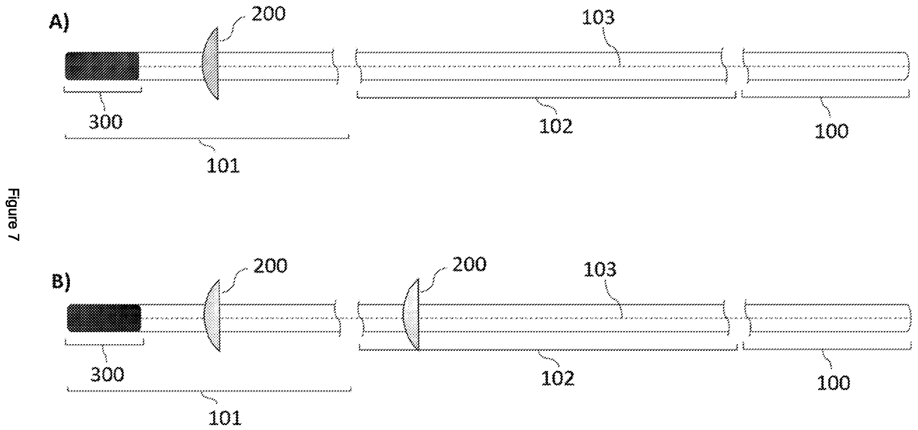

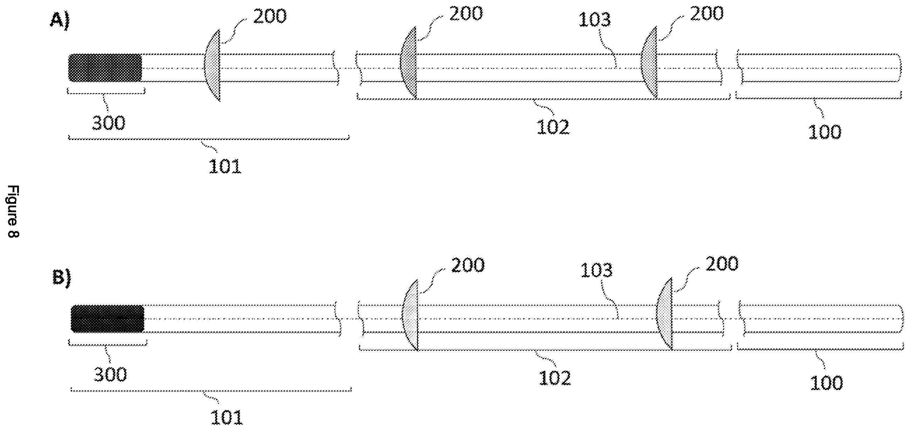

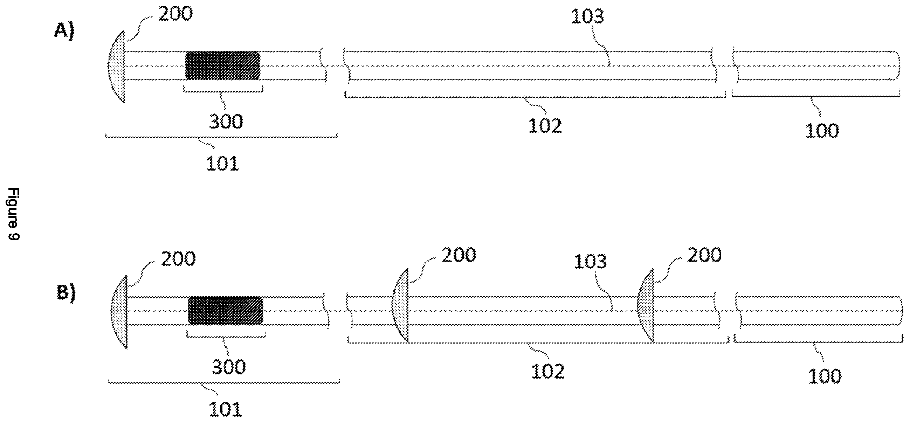

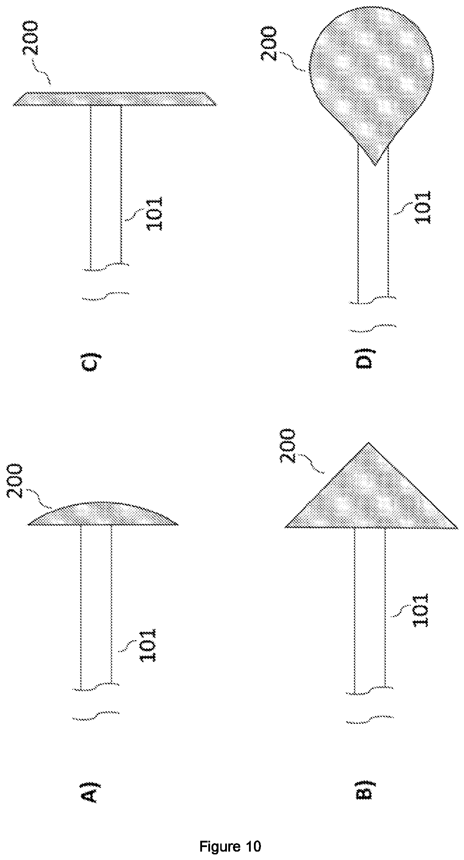

In some embodiments, the device of the invention comprises an element named in the frame of the present disclosure drag member **200**. As depicted for instance in Figures 7 to 9, the drag member **200** can be located distally along the device, but in some embodiments it can be located along the body **102;** additionally, more than one drag member **200** can be included in the device and distributed evenly or unevenly along the distal end **101** and/ the elongated body **102**. As shown in Figure 10, the drag member **200** can be designed and configured with different shapes, without departing from the inventive concept behind its function; for instance, the drag member **200** can be configured as a balloon, a parachute-shaped member, a pyramid-shaped member, a conical-shaped member, a cylinder-shaped member, a dome-shaped member, a ribbon-shaped member, a ciliated member and the like. Depending on its shape and size, the drag member **200** would facilitate the drag the flow directed device of the invention along a blood vessel with different speeds. In embodiments where more than one drag member **200** is present in the device, one or more combinations of size, shape, position along the device and/or material can be chosen for each and every member **200** to tailor the flow of the device.

Moreover, depending on the needs and circumstances, the drag member **200** can be configured to be deployable, either passively or actively, wherein for "actively deployable flow directed member" is herein meant that the drag member **200** can be deployed with the use of a mechanical/electrical/electromechanical system, while for "passively deployable flow directed member" is herein meant that the drag member **200** can be deployed by the blood flow itself based on the mechanical features and design of the member. As a way of example, a passively deployable drag member **200** can be configured as parachute-shaped member having several elongated members of a soft material connected between them through a thin film or membrane, opening and closing in turn based on the pumping of blood flood within a vessel. In another exemplary embodiment, an actively deployable drag member **200** can be configured as a balloon or an umbrella-like member capable of popping up from the very distal tip of the device, e.g. from an outlet **106** of a channel **104** running along the device, wherein the channel **104** comprises a mechanical/electrical/electromechanical system, or part thereof, designed to deploy the drag member **200** based on the user's needs. An actuator of said mechanical/electrical/electromechanical system can be located for instance at, or in close proximity of, the proximal end **100** of the device, and operatively connected to the drag member **200**. However, said actuator can be physically not in contact with the device, as in the case of a magnetically-actuated electromechanical system.

A drag member **200** can be built-in with the rest of the device or physically connected thereto. In terms of size, the drag member **200** lies in a range comprised between 1 and 500 $\mu$m for anyone of the three dimensions (width x length x thickness), in any combination, depending of course on the chosen design. Concerning the materials, a drag member **200** can comprise, or consist of, the same materials already listed elsewhere for the rest of the device, including polymeric, composite, metallic and/or oxide materials, as well as combinations thereof.

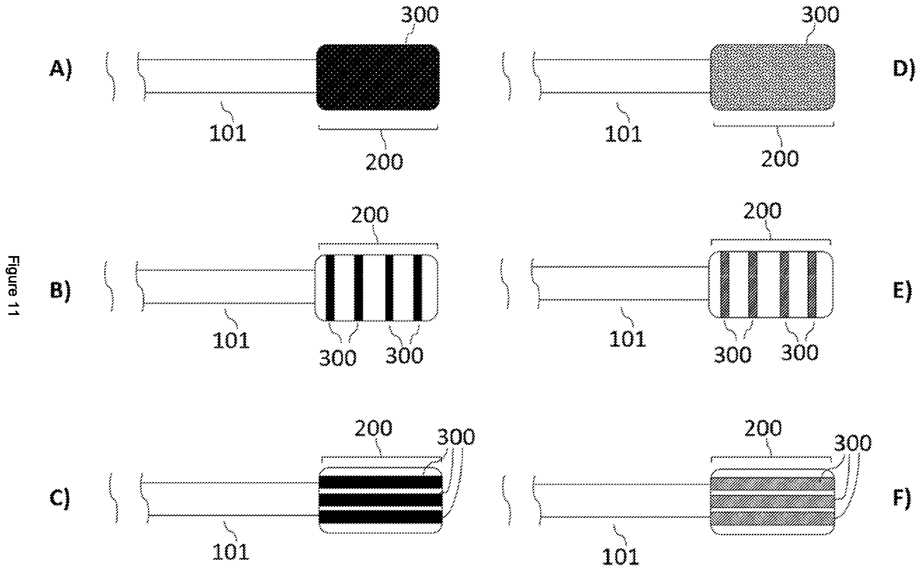

Moreover, as exemplarily depicted in Fig. 11, in one embodiment the drag member **200** can be distally located along the device and can include the at least one ferromagnetic area or structure **300.** For instance, a drag member **200** made of a soft polymeric material can be doped with ferromagnetic particles, ribbons, stripes and the like to create a built-in ferromagnetic area or structure **300.** This configuration has the advantage to easily coupling in a single element both functionalities (ferromagnetic features + enhanced drag function) without adding manufacturing burden.

Drag members can be distributed along the elongated body to ensure sufficient drag. Nanoribbos or unilateral denticles that are engineered on the surface of the elongated body can be activated and deactivated on demand depending on the deployment strategy. The activation of these structures increases the total drag force and distribute the drag on the entire length of the device.

Another aspect of the invention relates to a system configured to release a flow directed device according to the invention into a tubular structure, said system comprising:

a) the flow directed device as described herein; and
b) an insertion system **1000** comprising:

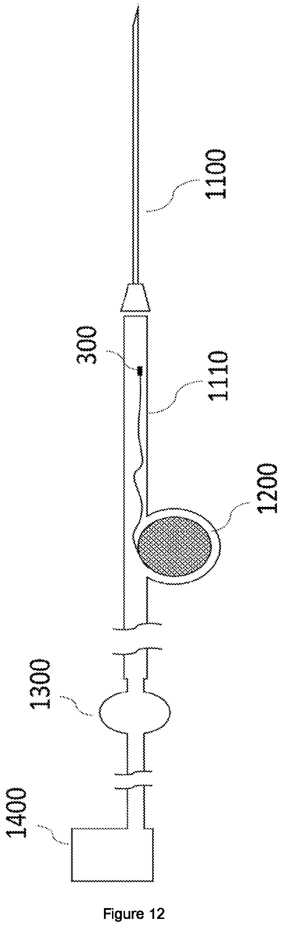

i) an insertion member **1100** for accessing a tubular structure;
ii) a releasing unit **1200** operatively connected with the flow directed device, the releasing unit **1200** being configured to gradually release the flow directed device without pushing it. One embodiment of this system is exemplarily depicted in Figure 12, which is optimized to release a flow directed device according to the invention into the bloodstream of a subject via a blood vessel.

In the non-limiting embodiment shown in Figure 12, the releasing unit **1200** substantially works as an actuator for the deployment of the device into the bloodstream of a subject. The releasing unit **1200** can be operated in any suitable way, such as through manual means, pneumatic means, hydraulic means, electromechanical means or computer-assisted means, as well as suitable combinations thereof as in case of a robotic system operably connected to the releasing unit **1200** or forming part of this latter. For instance, the releasing unit **1200** can include a guided slider mechanism or other means for enabling extension and/or retraction of the device such as a pivotable lever and fork, a user engageable pivotable lever-driven gear and rack attached to the proximal portion **100** of the device, a thumb wheel driven screw attached to proximal portion **100** of the device, a thumb driven rotatable pinion and rack attached to proximal portion **100** of the device, a simple hydraulic or pneumatic actuator of any type; a ball or lead screw actuator, or a low voltage electrical motor driven version of any of these. For enhancing the manoeuvrability of the entire device, the releasing unit **1200** or at least part of it can be included into a handle which can be easily used by a physician or otherwise an operator; in some embodiments, a handle can comprise activating/regulating knobs, buttons, levers or combinations thereof for an ameliorated and facilitated activation experience. All the possible electrical/electronic components of the releasing unit **1200** can be activated via embedded (rechargeable) batteries, wired connections to one or more external power supply, as well as in a wireless mode, for instance through (resonant) inductive coupling, (resonant) capacitive coupling, magnetodynamic coupling, ultrasounds and/or infrared radiation, by the simple implementation of solenoid antennas in any suitable position.

In a particular embodiment, the releasing unit **1200** comprises or consists of a spool around which the flow directed device is wound. In particular, the flow directed device of the invention is operatively connected with its proximal end **100** to the spool by any suitable mean known in the art such as adhesives, soldering, screwing and the like in a way as to be fixedly coupled to the releasing unit **1200.** In embodiments of the invention, moreover, the spool is configured as a removable cartridge. In such a configuration, part or all of the releasing unit **1200** can be easily removed or inserted together with the flow directed device in a single step, thereby facilitating and speeding up operations.

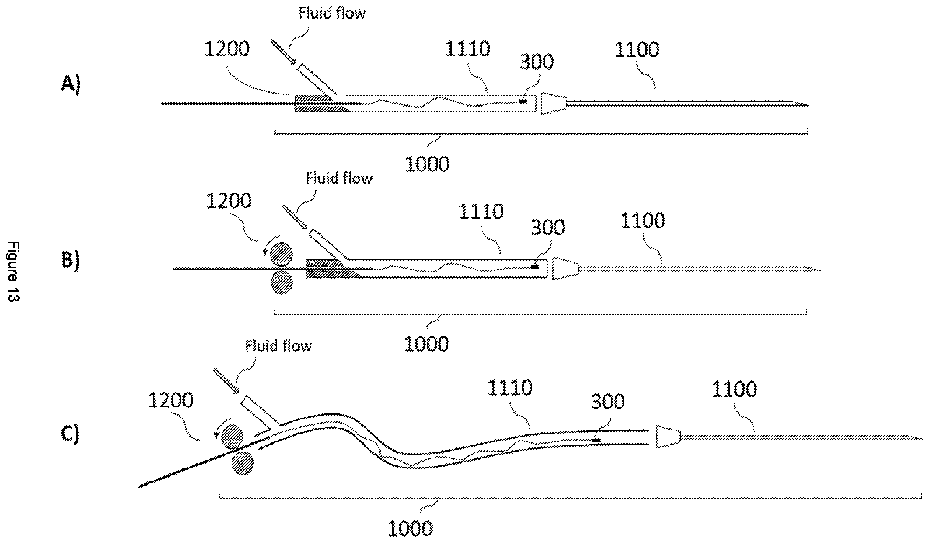

The insertion member **1100** is designed to access a tubular structure, such as for instance a blood vessel. Since the insertion system exploits fluid flow to regulate tension on the structures and transport the device, standard hypodermic needles and cannulas can be used as insertion members **1100** for e.g. percutaneous deployment. The minimal invasive intravascular insertion inside high-pressure arteries such as at the carotid level can therefore avoid open surgery and haemorrhagic complications. The insertion member **1100** can be operatively connected with an elongated member **1110** which might form part of the releasing unit **1200,** and which acts as a temporary "container" of the device before the beginning of the releasing operation (see Figure 13). For other tubular structures such as gastrointestinal or urinary ones, insertions tools such as trocars can be employed as aiding tool to access the subject's tubular body structure via laparoscopy.

Additionally, in preferred embodiments, the system of the invention further comprises a liquid reservoir **1400** operatively connected with the insertion member **1100** in a way to introduce a fluid such as a liquid into said insertion member **1100**. Actuation of a liquid flow is implemented through the fluidic connection and release of the liquid into the system in order to start the tensioning phase of the device before introduction into a tubular structure.

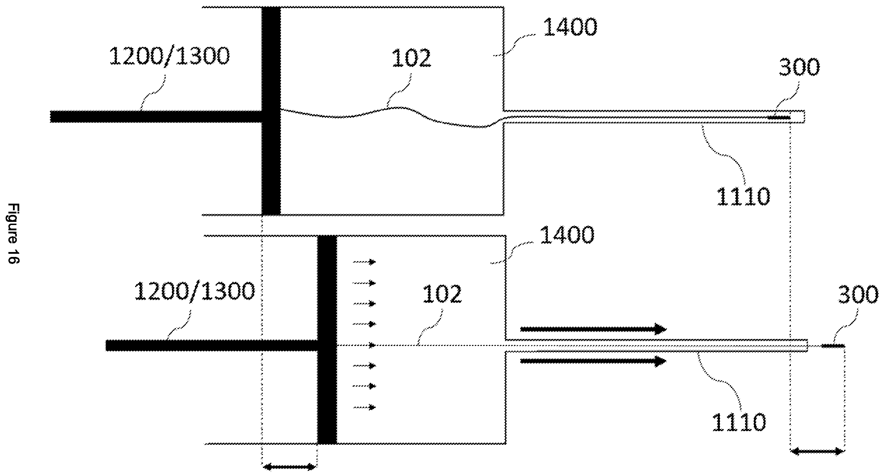

In some embodiment, the system further comprises pumping means **1300** operatively connected with said liquid reservoir **1400**. Said pumping means **1300** might be configured for instance as syringes, mechanical (micro)pumps, peristaltic pumps, haemostatic pumps and the like, or even passive pumping means, exploiting for instance capillary, osmotic or infusion forces. Pumping means **1300** and liquid reservoir **1400** are functional for the introduction and guidance of the device within a subjects' blood vessel, as will be described soon. In a further embodiment, as the one depicted in Figure 16, pumping means **1300** and the releasing unit **1200** might be embodied as a single element fluidically connected with the insertion member **1100**. In particular, the releasing unit **1200** can be inserted into a liquid reservoir **1400** and comprises or consists of a plunger or piston moveable along the reservoir **1400,** acting therefore *de facto* as an active or passive pumping means **1300.**

In view of the above, one aspect of the invention relates to a method for inserting a flow directed device into a tubular structure, such as a subject's tubular structure, comprising the steps of:

 a) providing a system as described herein;
 b) introducing the insertion member **1100** into the tubular structure; and
 c) operating the releasing unit **1200** in a way as to gradually release the flow directed device into the tubular structure without pushing it. Preferably, the method comprises a step of introducing, such as injecting, a liquid or fluid into the insertion system **1000**. In some embodiments, the method is performed for inserting a flow directed device into a subject's bloodstream via a blood vessel.

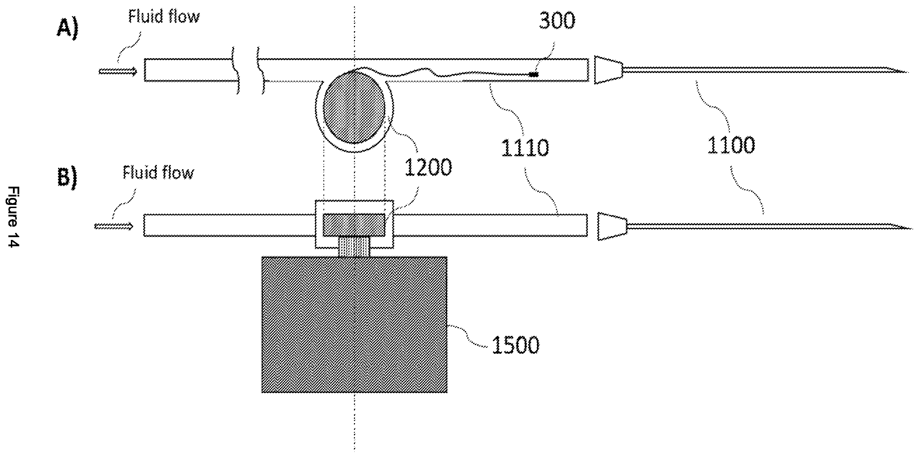

The insertion is performed by exploiting a fluid flow, such as a user-generated fluid flow, through a hydraulic circuit that regulates pressure at the operation side. For example, with reference to Figure 14, the device is winded around a motorized spool forming part of the releasing unit **1200,** whose rotation is controlled e.g. by a stepper motor **1500**. The deployment of the device into e.g. an artery is regulated by controlling the amount of a fluid flow, for instance a physiological solution flow, via the liquid reservoir **1400** acting as a source of physiological solution, preferably connected with pumping means **1300** , and the controlled release of the device by the spool **1200**. Given the extreme flexibility of the device, standard mechanical advancers would notably fail in feeding the floppy filament into a cannula because of mechanical instabilities such as buckling. Since the present insertion system **1000** exploits fluid flow to regulate tension on the structures and transport the device, standard syringe needles and cannulas **1100** can be easily used for percutaneous deployment.

As a way of example, one non-limiting method of use of the system of the invention involves performing a percutaneous or cutdown procedure to gain access to the vasculature of a subject. The insertion member **1100,** which in one aspect is preferably embodied as a hypodermic needle or a cannula, is introduced to reach a blood vessel and kept in place during the entire procedure. Upon introduction of the insertion member **1100** into a subject's body, in a first step a fluid flow of e.g. a physiological solution is gently released from the liquid reservoir **1400** through pumping means **1300,** exploiting an active pumping, hydrostatic pressure or capillary action, along the elongated member **1110,** which is in turn fluidically connected through its distal outlet to the proximal inlet of the insertion member **1100**. The fluid flow is selected based on the needs and circumstances, and is chosen based on the size, tensional strength and/or Young's modulus of the device. Typical fluid flows span in a range comprised between 0.1 and 10 ml/min, such as between 0.3 and 3 ml/min, with velocities typically around 10-30cm/s..

In a second step according to the method, the releasing unit **1200** is actuated in a way as to gradually release the flow directed device throughout the elongated member **1110**. In embodiments wherein the releasing unit **1200** comprises or consists of a spool, a stepper motor **1500** can be used to tailor the release and the deployment of the device. Of paramount importance, the fluid stream flowing within the elongated member **1110** keeps the device tensioned along the elongated member **1110** in such a way to avoid buckling of the device. The elongated member **1110** can be designed and configured to be of any size and shape, as long as it guarantees the safe deployment of the device; in some embodiments, the elongated member **1110** can be almost as long as the distal end **101** and the elongated body **102** of the device; in other embodiments, the elongated member **1110** can be shorter than the distal end **101** and the elongated body **102,** and can be shaped for instance as a serpentine or a corkscrew to keep the entire system more compact.

Moreover, the elongated member **1110** can be configured to be flexible by using a soft material for its manufacture.

In a third step of the method, once the device inserted into a subject's vasculature such as into an artery, the bloodstream drags the device along the artery without any push/pull operation from a physician. If needed, a fluid flow can be constantly kept or provided, from the liquid reservoir **1400** through pumping means **1300** to maintain the tensioning of the device within the subject's artery. Passive pumping means such as an intravenous (IV) bag positioned in order to exploit hydrostatic pressure would be perfectly suitable for the operation. Alternatively, a vascular bypass can be put in place exploiting the blood of a subject as a fluid source, so that the liquid reservoir **1400** becomes the patient itself. The advancement of the device along the vasculature is defined by the operator by actuating the releasing unit **1200**. Therefore, contrary to known device such as catheters or guidewires, the device of the invention can be advanced along the chosen path without pushing it, as the pushability of the device is close to zero.

One great advantage of such a mechanism of action is the speed of deployment. In the present method, the device has the potential to reach any region in a subject's body at the speed of blood, thereby reducing the duration of surgical operations and possibly limiting complications. Moreover, without being bond to any theory, it is believed that the inherent compliance of the device, as well as its completely passive, fluid-regulated mechanism of transportation, can protect the arterial lumen from damages such as escoriations and/or perforations.

Since the device advancement is entirely governed by the flow, the required level of expertise in guiding the catheter is almost null. If combined with advantage of virtually impossible punctures and wall friction, a robotic automatic catheterization can be achieved without imposing safety concerns generally arising from hard and heavy robotic systems. Moreover, the self-adaptation feature of the flow in the presence of obstacles (i.e. fluid follows the path with least resistance) would automatically change the device trajectory around the obstacle. As intuitive analogy, flow will always instantaneously solve an "hydraulic maze", provided that a flow exists from an inlet to an outlet. The ability to adapt upon geometrical conditions/perturbations while lacking of prior knowledge about the system, equips the device of the invention with some sort of "embodied intelligence", able to passively self-stabilize in new environmental conditions. Also, there is no need for variable stiffness control as the flow governs over the shape of the device according to the vessel geometry.

Still another object of the invention relates to a method for guiding a flow directed device into a tubular structure, such as ones present in a subject's body, said method comprising:

a) providing a system according to the present invention;

b) providing means **2000** for inducing an external magnetic field **3000** in proximity to said system;

c) inserting a flow directed device into a tubular structure according to the method as previously described; and

d) operating said magnetic field inducer **2000** in a way as to provide an external magnetic field **3000** capable to deflect the distal end **101** of the device in correspondence of a target point of the tubular structure. In some embodiments, the tubular structure is a blood vessel, including branched vessels or networks. For "target point" is herein meant a point or portion of the tubular structure such as turns, branches, bumps (e.g. aneurysms or stenosis) and the like. Means **2000** for inducing an external magnetic field might be for instance at least one or more, such as two, four, six, eight or more electromagnets.

The method for guiding a flow directed device into a subject's body according to the invention exploits both the method for inserting a flow directed device into a tubular structure, such as a blood vessel, and the magnetic characteristics of the device of the invention. Once the device introduced, it can be guided through the application of a uniform or gradient external magnetic field **3000** influencing the off-axis deflection of the distal end **101** by acting on the ferromagnetic area or structure **300**.

In a non-limiting embodiment, as exemplarily depicted in Figure 15, the deflection of the distal end **101** can be computer-controlled by permanent magnets or electromagnets **2000** assembled e.g. in a Helmholtz configuration in order to generate a spatially homogeneous magnetic field **3000** and provide magnetic torque without any application of pull-push forces. The magnetic torque exerted on the ferromagnetic area or structure **300** deflects the distal end **101** of the device, which in turn interacts with the fluid flow utilizing changes in the drag forces to steer the device. Unwinding the device after e.g. magnetically positioning the distal end **101** at a bifurcation **801,** allows continuing the advancement of the device along the chosen path of the tubular structure **800**. By tuning the size and flexibility of the magnetic tip of the device and modulating the strength of the applied magnetic field **3000,** the exact path the device will follow under the influence of the fluid flow can be possibly even determined *a priori*.

Additionally or alternatively, the magnetic field **3000** can be obtained even via a single coil **2000,** and the obtained magnetic field **3000** can be non-spatially uniform (e.g., a gradient magnetic field) in order to pull the device along e.g. blood vessels in case of a small or absent blood flow (e.g. in veins). This configuration enhance the universality of use of the system of the invention in scenarios where a push-pull operation might be needed.

As it will be evident, the devices, systems and methods described in the present disclosure can be used for the treatment, diagnosis, monitoring and/or prevention of a pathological conditions of a subject, preferably a human being, for which the device, system and/or method of the invention might be beneficial.

In a non-limiting example, the devices, systems and methods described in the present disclosure can be used for the treatment, diagnosis, monitoring and/or prevention of a disorder of the Central Nervous System (CNS) in a subject, preferably the brain and its associated structures.

In some embodiments, the devices, systems and methods of the invention can be used to treat or monitor a nervous tissue such as a nerve forming part of the sympathetic nervous system. The devices, systems and methods of the invention can be particularly useful in some conditions in which a sympathicotonic effect (a stimulated condition of the sympathetic nervous system in which there is increased tonus of the sympathetic system) appears, such as a vascular spasm (e.g. cerebral vasospasm) or elevated blood pressure conditions as congenital, chronic or idiopathic hypertension. Accordingly, in one embodiment, the nervous tissue can be electrically modulated, such as stimulated, or monitored by the device of the invention. This effect can be obtained by the introduction of the device into e.g. a segment of the internal carotid artery such as the cervical section (C1), the petrous section (C2), the lacerum section (C3), the cavernous section (C4), the clinoid section (C5), the ophthalmic section (C6) and the communicating section (C7), to modulated e.g. the stellate ganglion, the inferior cervical ganglion, the middle cervical ganglion, the superior cervical ganglion, the carotid body, the cavernous plexus, the ciliary ganglion and the pterygopalatine ganglion. These embodiments are preferred in case the pathological condition to be treated by device, system and methods are e.g. cerebral vasospasm, as Raynaud's disease or Complex Regional Pain syndrome.

In alternative embodiments, treatment-refractive hypertension, i.e. high blood pressure not controlled by medication, can be treated by device by performing electrical ablation inducing renal denervation, wherein nerves in the wall of the renal arteries are ablated by applying electrical pulses to the renal arteries, causing a reduction of sympathetic afferent and efferent activity to the kidney and blood pressure decrease.

The devices, systems and methods of the invention of the invention can even be possibly used for the treatment of other pathological conditions involving an abnormal activation of a portion of the sympathetic nerve trunk, through e.g. ablation of relevant nerves anywhere in either of the two sympathetic trunks. Examples of said pathological conditions are Raynaud's disease, hyperhidrosis, Moyamoya disease, migraines, hyperactive bronchial tubes, long QT syndrome, social phobia or anxiety. Further nervous structures can be treated/ablated such as pudendal nerves, mesenteric ganglia, celiac ganglia, coccygeal ganglia, cervical ganglia, splanchnic nerves, or glossopharyngeal nerves for pain (such as cancer-derived or chronic pain) management, treatment of premature ejaculation and other conditions. In some alternative embodiment, Vagus Nerve stimulation

(VNS) can be used for the treatment of intractable epilepsy or treatment-resistant depression, while Vagus Nerve blocking can be used to treat obesity. Furthermore, the device can be used in association with other devices and systems to overcome the secondary effects of certain invasive therapies, such as in case of a catheter-induced coronary artery spasm upon coronary catheterization.

The present invention can also be used for fast and reliable access to deep malformations spots in a subject (e.g. arteriovenous malformations, AVM) or fistulas (e.g. arteriovenous fisulas, AVF). The ability to propell by the fluid stresses facilitates trackability and accessibility in the presence of difficult bifurcations. This aspect is particularly useful in situations where the target vessels are barely captured with fluoroscope images.

Another advantage of flow-directed navigation of ultraflexible micrometer probes is the ability to circumnavigate the arteriovenous system, especially in the presence of major anastomoses. Reaching the target location through arterial vessels and subsequently downstream navigating in the veins, allows tracing the optimal trajectory path for the retrograde advancement of standard venous catheters system. Moreover, the ability to retract the flow-directed probe can be used to mechanically pull the distal portion of a catheter (or guidewire) towards the target region.

The ability to navigate without direct human intervention and with minimal risk of perforation or dissection enables the introduction of teleoperated or autonomous robotic platforms. While standard catheterization techniques require real-time force feedback for safe operation, the device and system of the invention can be safely operated with only visual feedback or with no feedback and trial and error. Forces created by the magnetic field can be maintained below 1 mN, which precludes damage to soft tissue. In addition, the absence of pushing forces nullifies iatrogenic damages induced by the insertion. The practitioner can steer the device using a robotic teleoperation system outside the operation room, eliminating the risk of X-ray exposure to the practitioner. Alternatively, for a given map of the vasculature, the practioner can devise a navigation strategy based on how to release the device and apply the magnetic field, and this plan can be converted to a computer program that can be played during the operation.

Fully automated operation can be realized by a computer program that is equipped with algorithms for planning and navigation. The computer program can use the fluoroscope images to control the release of the device and the magnetic field and guide the device toward a target region. The program can further implement state-of-the-art algorithms based on machine learning and artificial intelligence.

In still other embodiments, the devices, systems and methods of the invention can be used for electrically sensing a physiological parameter or stimulating an electrical response in a nerve of a subject and/or a pharmacological activity. The sensing or stimulation activity can be performed in the frame of a therapeutic or preventive (e.g. for diagnostic purposes) medical treatment in a subject.

## EXAMPLES

### Design and operation of a $\mu$probe-insertion device

Unlike conventional endovascular catheters, ultra-flexible and ultra-lightweight filaments cannot simply be pushed into a stream because axial compressive loads will no longer be effectively transmitted due to bending of the $\mu$probe. For this purpose, it was developed a hydromechanical insertion system that seamlessly couples with the vasculature and keeps a flexible $\mu$probe under tension during the entire operation. The proximal extremity of the $\mu$probe was attached to a rigid rod (i.e. plunger) that slid across a sealing gasket at the rear end of a custom-made syringe barrel, and the motion of the rod was controlled by a linear positioner. Gentle pulling of the rod ensured proper loading of the $\mu$probe into the barrel. Saline solution was pumped inside the system through an intake, applying tensile stress to the relaxed filament. Percutaneous cannulation in conjunction with controlled perfusion was performed for the fast and versatile deployment of the $\mu$probe (Figure 17). The axial movement of the rod determined the portion of the $\mu$probe that was released into the target vessel. The hydrodynamic pulling forces ensured effortless passage of $\mu$probes into the cannula in the absence of pushing forces. Upon introduction of the $\mu$probe into the vessel, viscous stresses applied by the physiological blood stream dominate the propulsion and the perfusion may be terminated.

### Fluid-structure interactions in ultra-flexible $\mu$probes

The deformation and passive deployment of the $\mu$probe was first studied in a nonuniform viscous flow with curved streamlines. Flexible electronic devices fabricated by depositing conductive elements on thin polymer substrates offer a versatile route for the development of smart $\mu$probes. The inventors manufactured 4 $\mu$m thick polyimide ribbons with 200 $\mu$m width as generic structures for the study of fluid-body interactions under the influence of flow. Coating the complete surface of the ribbon with a 100 nm thick gold film exaggerates the stiffness of functional $\mu$probes with patterned electrical circuits.

Cylindrical magnetic heads with varying sizes (diameter on the order of 200 $\mu$m) were fabricated from a hard-magnetic elastomer composite using a moulding process. The experiments were performed inside biomimetic vascular networks made from photopolymer or elastomer using 3D printing and sacrificial moulding, respectively. A Newtonian fluid matching the viscosity of blood was pumped into the channels. The inventors regulated the average fluid velocity, $\overline{u}$, according to the size of the vessels to stay in the physiologically relevant regime.

A tortuous fluidic scenario was realized to replicate challenging vascular trajectories as shown in Figure 18a. As soon as the $\mu$probe was fed into the channel using the insertion device, fluid flow started to pull on the structure and kept it under tension at all times. The $\mu$probe was fed by moving the plunger forward and the filament perfectly followed the central streamline in the linear portion of the channel. The viscous stresses bent and transported the $\mu$probe through each high-curvature turn to the target location. For the chosen geometric and flow parameters (i.e. curvature up to 0.8 mm-1 and $\overline{u}$ = 20 cm·s-1) the maximal attainable velocity of the $\mu$probe was 2.8 cm·s-1, which corresponded to approximately 0.15 $\overline{u}$. Intuitively, faster advancement can be achieved at higher fluid velocity due to the increase in viscous stresses on the $\mu$probe. Likewise, straighter channels allow faster deployment, pushing the maximum attainable deployment speed closer to the flow velocity. To demonstrate the instrumental role of the viscous stresses in the advancement process, inventors temporarily turned off the pump and kept pushing the $\mu$probe. The structure immediately lost tension along with hydrodynamic lift upon removal of the flow. With further pushing, the contact between the walls of the channel and the magnetic head generated mechanical instabilities such as buckling (Figure 18b). The structure unfolded and regained its straight configuration as soon as the pump was turned on, regardless of the deformed shape of the $\mu$probe.

The sustained tension on the $\mu$probe provided by the viscous stresses differentiate the proposed deployment system from conventional strategies. Standard push-based endovascular devices rely on the outward wall contact to be able to transmit the axial force along the structure in non-linear trajectories (Figure 18c, left). The result is an important increase in friction and normal forces on the walls that would ultimately overcome the proximal insertion force and lead to advancement arrest or potentially to blood vessel wall perforation. Oppositely, in flow-driven navigation, the contact points are limited to the inward wall of the curve due to the experienced tensile regime (Figure 18c, right). The discrete contact regions, in combination with the distributed drag force, allows maintaining a ratio larger than one between the total drag force and friction force along the entire trajectory and therefore ensuring uninterrupted advancement (Figure 18d). The tip of the $\mu$probe followed almost identical trajectories while it was being pulled forward by the flow or pulled back by the insertion device. One important consideration for the retraction process is the friction at corners with high curvature. The structure must be ultra-flexible yet inextensible for effective and safe retraction through tortuous paths. Since the position of the $\mu$probe depends on the flow along its length, the $\mu$probe is not always aligned with the local streamlines. For example, when the flow has curved streamlines, the filament does not align with the flow, but rather it crosses the streamlines.

*Adaptive transport of $\mu$probes in complex vessel phantoms*

Using elastohydrodynamic coupling for propulsion enables enticing opportunities for $\mu$probes navigating inside highly curved, structured, or dynamically changing environments. It was explored whether the elastic body could continuously morph in accordance with the geometry of the channels by testing the devices in a phantom with a braided channel forming a 3D knot with curvature as high as 0.25 mm-1 (Figure 19a). The $\mu$probe effortlessly completed the track at a deployment velocity of 4 cm·s-1 under the control of the fluid flow with $\overline{u}$ = 30 cm·s-1.

The rapid pursuit of streamlines without any external manipulation set the ground to test whether the $\mu$probes can pass through occlusions mimicking extreme vascular stenoses. Inventors fabricated a channel with two internal occluding walls where the only passage was provided through a circular window with a diameter twice as large as the size of the magnetic head. Computational Fluid Dynamics (CFD) simulations visualized the 3D streamlines inside the channel and quantified the distribution of flow velocity (Figure 19b). The $\mu$probe autonomously navigated through both apertures while the kinematics of the motion followed the flow field imposed by the occlusions (Figure 19c). The $\mu$probe moved as fast as 2.8 cm·s-1 at $\overline{u}$ = 20 cm.s-1 .

*Magnetic actuation of the tip for controlled bending of the $\mu$probe*

While the translational motion of the $\mu$probe is controlled by the drag forces, selection of a specific path requires the application of external torque. It was observed that the transversal tip position of the $\mu$probe, $y_{tip}$, right before entering a bifurcation is a reliable indicator for the direction the $\mu$probe is going to take (Figure 20a). The inventors hypothesized that by rotating the head of the $\mu$probe and positioning the tip at the correct location with respect to the stagnation streamline (white dashed line), they could harness the current in this new position (streamlines) to have the $\mu$probe pulled into the downstream vessel. The $\mu$probe would then be transported to the next bifurcation autonomously in the absence of external manipulation and the same protocol would be applied to select a new trajectory. It was decided to use magnetic actuation to apply torque due to the ease of generating uniform magnetic fields, scalability arguments, and medical compatibility.

It was systematically studied the effects of the magneto-elastohydrodynamic coupling on ytip to develop a robust strategy for navigating $\mu$probes. Inventors fabricated a representative $\mu$probe with a cylindrical magnetic head ($\phi$H = 350 $\mu$m and LH = 1 mm) and used this prototype for the following characterization experiments, unless stated otherwise. Upon application of the magnetic field, the head rotated in order to align with the direction of the field, which changes the distribution of hydrodynamic forces and elastic stresses, leading to a new equilibrium configuration (Figure 20b). In the first set of trials, the $\mu$probe was placed in the middle of a channel at $\overline{u}$ = 30 cm□1 and subjected to varying magnetic fields (Figure 20c). At low $B$, the lift force acting on the structure moved the entire $\mu$probe to the upper half of the channel in the opposite direction of the field. This steering mechanism closely mimics the motion of wakeboards and reaction ferries. At $B \geq 8$ mT, the $\mu$probe started to contact the upper wall (arrow), which constrained the lift-induced upwards motion of the structure and served as a support point to further bend the distal end towards the direction of the magnetic field.

At higher B, magnetic head rotated further, which resulted in the entry of the $\mu$probe tip into the bottom half of the channel. The effect of magnetic actuation was incorporated into a computational model by discretizing the flexible head and applying magnetic moment at the corresponding nodes. It was tuned the magnetization value to match the equilibrium shape at a certain $B$ and $\overline{u}$, and used the same calibrated value for the following simulations.

Next, the inventors performed a velocity sweep at $B$ = 8 mT. With increasing $\overline{u}$, the contribution of the hydrodynamic forces to the final shape also increases, resulting in a straightening of the $\mu$probe. As a result, the tip eventually moved from the bottom to the upper half of the channel (Figure 20e). The overlay of the equilibrium $\mu$probe shapes under varying $\overline{u}$ and B summarized the competing effects of hydrodynamic forces and magnetic torque on $y_{tip}$ and the steady-state shape of the $\mu$probe.

*Controlled navigation of $\mu$probes in fluidic networks*

After establishing a method for effective tip positioning under flow, the inventors tested the success of steering in the presence of bifurcations. The $\mu$probe was fed into a channel that splits into two symmetrical daughter vessels with $\beta$ = 40° and $\overline{u}$ = 30 cm·s-1. Two distinct navigation strategies for selecting daughter vessels were found. In Controlled-Lift (CL), the rotation of the head drifts the structure towards the selected streamline by hydrodynamic lift (Figure 21 a-b). Navigation based on CL is particularly appealing for situations where contact with the walls must be minimized, magnetic torque is limited, or fluid velocity is relatively high. On the other hand, in Controlled-Heading (CH), magnetic torque imposes the head pose while working against hydrodynamic forces (Fig 5b). CH was generally accompanied with the μprobe contacting the channel wall, even though the success of the navigation did not rely on this boundary effect. While the forward velocity of the μprobe (i.e. the velocity at which the μprobe is released) was set to 4 mm·s-1, the navigation strategies worked at velocities as high as 0.2 $\overline{u}$. The inventors recapitulated the transport of the μprobe with a computational model by iteratively moving the whole structure forward along the longitudinal axis. The simulation results matched major empirical observations - CL was achieved by exploiting lift to reach the target streamline and enter the daughter vessel at relatively low *B* (Figure 21c). CH was successfully executed at higher B while the simulated μprobe contacted the walls at similar locations (Figure 21d).

After recording the results of several navigation trials at different magnetic fields, it was possible to discriminate two separate regimes favouring navigation based on CL and CH. Figure 21e shows a representative plot of the dominant navigation strategies for varying Bat $\overline{u}$ = 26 cm·s-1 and β = 40°. A phase diagram was created by repeating this characterization process at different flow rates (Figure 21f). In the phase diagram, every line corresponds to a plot that is in the form of Figure 21e, binarized with an 80% confidence. Next, concatenated diagrams were generated by stacking phase plots for different β (Figure 21g) and head geometry (Figure 21h). All results showed that the CL regime is predominant with increasing $\overline{u}$, while CH navigation had better success rate at bifurcating angles above β = 40° for a given velocity. Increasing the aspect ratio of the magnetic head in μprobe design promoted CH over CL due the restricted space for the motion of the head. Decreasing the cross-sectional area of the channel from 2 x 2 mm$^2$ to 1 x 1 mm$^2$ completely precluded navigating the μprobe using the CL method with the chosen design and mechanical properties. Decreasing the magnetic head size and/or the bending stiffness of the μprobe will allow navigation using the CL method in sub-mm vessels.

Although the inventors showed navigation in a single plane, magnetic steering can easily be extended to 3D trajectories. The inventors used the 3D design of the phantom to coordinate the application of the magnetic field with the release of the μprobe during navigation. Real-time closed-loop feedback control in the absence of a map requires 3D imaging of the vessels and the probe head. In principle, controlled application of magnetic torque only when the μprobe approaches bifurcations would suffice for reaching target locations. Magnetic continuum devices provide a rich repertoire of actuation modes that can generate propulsion also inside stationary fluids: by actuating the head with time-varying magnetic fields, entry into hydrodynamically unfavourable vessels can be facilitated. With further miniaturization, navigation inside microfluidic channels as small as 100 μm in width became feasible. μprobes with a size of 25 μm x 4 μm and a 40-μm diameter magnetic head was successfully transported in less than two seconds. Using μprobes within a similar size range, distal microvasculatures in the body may be reached, a feature that is currently impossible to achieve up to the inventors' knowledge.

Finally, the implemented navigation strategy is compatible with the deployment of multiple μprobes. The inventors iteratively steered four μprobes to pre-determined target locations **in** a phantom with three bifurcations. The profile of the flow around the existing μprobes autonomously creates alternative paths for the incoming μprobes. Thus, the number of μprobes that can be simultaneously deployed is only limited by the relative size of the probe with respect to the vessel diameter.

*Local characterization of flow using electronic μprobes*

After establishing the navigation strategy, it was explored the possibility to dynamically record physiological parameters such as electric potential, temperature, or flow characteristics using μprobes. Polyimide substrate was used to fabricate the main chassis while gold and platinum strips were deposited to form electrodes and electrical circuits, respectively. As a proof of concept, a μprobe that uses convective heat transfer was engineered to measure the characteristics of local fluid flow. Upon injection of current into the heater circuit, the generated heat is transferred to the sensor circuit at a rate determined by the velocity profile of the fluid. The fabricated electronic μprobes consisted of a 0.1 mm x 2 mm heating element and a 200 μm x 500 μm sensor component, positioned 50 μm apart from each other (Figure 22a).

Conventional flow sensors that are based on heat transfer operate either in continuous mode or pulsed mode. In continuous mode, the change in the sensor resistance with the flow is monitored. As a result, this measurement requires high Signal-to-Noise Ratio (SNR), making it challenging to be implemented on miniaturized devices. The flow sensors of the invention were operated in pulsed mode, by measuring the Time-Of-Flight (TOF) between the injection of current to the heater and the detection of peak resistance at the sensor. This method requires high sampling rate, which does not pose a trade-off with miniaturization.

The input to the heater was a 10-ms current pulse with 4 mA peak value and the sensor resistance was recorded at 5 kHz sampling rate (Figure 22b). A single temporal readout was acquired within 150 ms under physiologically relevant fluidic regimes. The speed of sensing allowed real-time interrogation of the flow during navigation. The inventors performed a series of measurements at the wall of a channel with 2 x 2 mm$^2$ cross-sectional area at varying fluid velocities. The data showed an exponential decrease in the TOF with increasing $\overline{u}$ (Figure 22c). Notably, it was found that the measurements were strongly influenced by the position of the μprobe as a manifestation of the varying flow profile. As a result, the TOF was calibrated with respect to the wall shear stress, $\tau_{wall}$. The wall shear stress was computed from the fully-developed flow profile, which was extracted using the Fourier sum approach.

To validate the calibration curve at different channel geometries and flow conditions, the inventors made on the fly measurements of $\tau_{wall}$ while being navigated within structured channels. The µprobe was deployed into a stenotic region where the cross-sectional area of the channel was reduced from 2x2 mm$^2$ to 1x1 mm$^2$ (Figure 22d). Empirical data recorded at the pre-stenotic and stenotic regions varied only by 2.3% and 13.9% from the analytically calculated values (Figure 22e). The relative increase in $\tau_{wall}$ was approximately 8-fold as expected from the relation $\tau_{wall} \propto \overline{u}/h$, where $h$ is the height of the channel. As a final demonstration, it was verified that magnetic actuation and bending of the filament did not interfere with the flow measurements. The µprobe was navigated in a channel with two branches that had different cross-sectional area (Figure 22f). The µprobe was successfully positioned at both branches, and the shear stress measurements were close to the calculated values with 4.6%, 8.5% and 9.0% error, respectively. The repeated measurements performed at different locations and with 3 different µprobes showed reliable measurement and manufacturing.

*Ex vivo demonstration using microscale µprobes and µcatheters*

To test the feasibility of the navigation of the µprobes under physiological conditions, the inventors performed ex-vivo tests on perfused rabbit ears (Figure 23a). The favourable transparency of the tissue allows standard camera imaging of µprobes without requiring advanced medical angiography systems. Moreover, the subcutaneous position of the vessels enables percutaneous insertion of the µprobes with our hypodermic insertion device. The inventors first injected a blue ink into the central artery to map the arterial system (Figure 23b). Four target locations were chosen (denoted by capital letters) and three layers of bifurcations were marked (i, ii and iii). The ear was perfused with a saline solution at a constant flow rate between 30 - 90 µL·s-1. A µprobe with a $\phi_H$ = 150 µm and $L_H$ = 3 mm magnetic head and 4 µm x 250 µm body cross-section successfully reached all target locations through CH navigation under three seconds with an average advancement velocity of 1 cm·s-1 (Figure 23c). The navigation speed can be ultimately increased by efficiently synchronizing the applied magnetic field with the release of the µprobe. The µprobe entered all the physically fitting vessels, with no apparent friction arising from vessel walls. In fact, ex vivo tests revealed excellent lubrication capabilities compared to in vitro experiments.

Slender filaments in different forms and material composition may further extend the capabilities of the proposed concept of flow-driven navigation. Conventional catheters are manufactured as tubes that enable injection of contrast agents, stents and embolization agents, such as glues or microwires, or even allow collection of blood clots. Motivated by these capabilities, the inventors fabricated elastomer microtubes using a thermal polymerization technique. A tubular magnetic head was glued to the distal end of the flexible tube to complete the µcatheter. This protocol allows fabricating Polydimethylsiloxane (PDMS) µcatheters with outer diameter as small as 120 µm and several cm-long (Figure 23d). Upon perfusion through the insertion system, the hydrodynamic forces effortlessly transported the flexible µcatheter inside the vessels of the rabbit ear, and magnetic steering allowed the device to navigate through bifurcations. To demonstrate the potential for endovascular embolization or targeted drug delivery, it was temporarily stopped the perfusion and injected a blue dye at various locations within the vascular network (Figure 23e). The diameter of the rabbit ears, where the dye injection was performed, were estimated from the evacuation speed of the dye upon re-activation of the perfused flow. Assuming equal flow distribution at the first two bifurcations and no other prior bifurcation, the inventors estimated the diameter of the artery at the point D to be 300 µm. Experimental observation corroborated with this estimation; the µprobe in Figure 23c got stuck at point D, indicating a vessel diameter in the order of the largest feature of the µprobe (width = 250 µm).

*Experimental Platform.*

The experimental setup consists of an electromagnetic manipulation system, a linear positioner, and a fluid circuit (Figure 24). Homogenous magnetic fields are generated within a volumetric space of 94 x 53 x 22 mm$^3$ with maximum magnetic field strength of 50mT, 60mT and 85mT, respectively. The current flowing through the coils is provided by three sets of power supplies (S8VK 480W, Omron and SM 52-AR-60, Delta Elektronika) and servo controllers (Syren25 and Syren50, Dimension Engineering), which are modulated via an analog/digital I/O PCi Express board (Model 826, Sensoray). The off-the-shelf motorized stepper-motor linear positioner controls the forward and backward motion of the connecting rod and, as a result, the position of the µprobes. Teleoperation of µprobes was performed using a 3D mouse (3DConnexion) and keyboard. The fluid flow was provided by a peristaltic pump (LP-BT100-2J, Drifton). The oscillations in pressure were dampened using a Windkessel element. A solution of 42.5 wt% of glycerol (Sigma-Aldrich) in deionized water was used as a blood analogue. Visualization of the µprobes inside the phantoms was performed using CMOS camera (acA4024-29uc, Basler). An illumination system was built from smartphone backlights.

*Fabrication of electronic μprobes.*

Device were fabricated using standard micro-fabrication techniques. Briefly, generic μprobes have been prepared by spin-coating on a 4-inch Si wafer a 4 μm-thick layer of polyimide (PI2610 Hitachi Chemical DuPont MicroSystems GmbH). A positive photoresist (AZ1512, 2 μm exposed by Heidelberg Instruments MLA150, 405 nm and 104 mJ.cm-2 and developed by AZ 726 MIF developer for 45s) has been used as mask for sputtering (Alliance Concept AC450) a 100 nm-thick layer of gold in stripes of 250 μm width and 9 cm length, followed by lift-off in acetone. The μprobe borders have been then laser cut (Optec MM200-USP) and detachment from the wafer has been done manually. The fabrication of the flow sensors involves the sputtering of titanium and platinum layers serving for adhesion and electrical conduction, respectively. To add the two serpentines, designated as temperature sensor and heater, another 25 nm-thick layer of platinum has been sputtered over the wafer, after surface activation by argon plasma (Alliance Concept AC450). The shapes of the traces and serpentines were defined using spin-coating and removal of positive photoresists (AZ9260, 8 μm and AZ1512, 2 μm). The electrical circuits were sandwiched between polyimide layers.

The magnetic head for all μprobes was fabricated from a composite of PDMS and Neodymium Boron Iron (NdFeB) microparticles with an average diameter of 5 μm (Magnequench, Germany), mixed at a mass fraction of 1:1. The structures were cured at 65° C in the oven in a custom-made mould. The size of the cylindrical magnetic heads varied from 40 μm to 350 μm in diameter and from 500 μm to 3 mm in length. Magnetization was performed using an impulse magnetizer (Magnet-Physik) at a field strength of 3'500 kA.m-1. The magnetic head was attached to the distal end of the Kapton ribbon using epoxy and manual positioners (Thorlabs). The electrical measurements were performed using a sensitive dual-channel source measure unit (Keysight B2902A).

*Fabrication of μcatheters.*

A 40 μm-diameter tungsten wire (Goodfellow) was immersed in PDMS (10:1 ratio) and heated with electrical current at a density of 320 A·mm-2. Thermal dissipation provided by Joule's heating ensured local polymerization around the wire. The thickness of the PDMS μcatheter was determined by the time of polymerization. The wire coated with the polymerized PDMS was then immersed in an acetone bath and vigorously agitated to remove uncured PDMS. After completing curing in an oven at 65° C for 2h, the tungsten wire was first gently and mechanically disengaged from the PDMS coat using tweezers. Next, to apply a distributed load, the PDMS μcatheter was sandwiched between two sheets of PDMS and the tungsten wire was gently pulled out while applying pressure on the PDMS sheets. A tubular magnetic head was bonded to the distal end of the μcatheter using PDMS as a glue and a tungsten wire to prevent clogging.

*Ex vivo experiments.*

Navigation experiments were performed with ex vivo Rex rabbit (weight between 2.8 kg and 3.5 kg) ears immediately following their delivery from a local butcher. The ears were perfused with Phosphate-Buffered Saline (PBS) solution 1X (Sigma Aldrich) in the central artery shortly after excision. The μprobes were introduced into the central ear artery using the insertion device and a 21 G hypodermic needle. A 3.10-3 w/v% of Pluronic-F127 solution (Sigma Aldrich) was added to the PBS solution to avoid adhesion of the PDMS tubes to the barrel of the insertion system. Mean perfusion flow was maintained at a rate of 90 μL·s-1.

While the invention has been disclosed with reference to certain preferred embodiments, numerous modifications, alterations, and changes to the described embodiments, and equivalents thereof, are possible without departing from the sphere and scope of the invention. Accordingly, it is intended that the invention is not limited to the described embodiments, and be given the broadest reasonable interpretation in accordance with the language of the appended claims.

**Claims**

1. A flow directed device adapted to be introduced in a tubular structure of a body of a mammal, such as a blood vessel, said device comprising a proximal end (100), a distal end (101) and an elongated body (102) in between defining a longitudinal axis (103), and a ferromagnetic area or structure (300) located on said distal end (101) capable of deflecting the distal end (101) when subjected to an external magnetic field, **characterised in that**, at least said distal end (101) is configured as a ribbon.

2. The flow directed device of claim 1, wherein at least the distal end (101) has a flexural rigidity comprised between about $10^{-10}$ and about $10^{-14}$ Pa·m-4.

3. The flow directed device of any one of claims 1 or 2, wherein at least the distal end (101) and the elongated body (102) have an elastic modulus comprised between about 100 kPa and about 10 GPa, preferably between 500 kPa and 5 GPa.

4. The flow directed device of any one of the previous claims, wherein the cross section perpendicular to the longitudinal axis (103) has a dimension comprised between about 500 nm and about 30 μm,

between 1 and 10 $\mu$m, between 2 and 20$\mu$m or between about 1 $\mu$m and about 5 $\mu$m and wherein the width is comprised between about 10 $\mu$m to about 500 $\mu$m, such as between 30 and 300 $\mu$m, between 50 and 200 $\mu$m or between 50 and 100 $\mu$m.

5. The flow directed device of any one of claims 1 to 3, wherein the device has a tubular shape with diameters ranging from about 500 nm to about 500 $\mu$m, such as between about 1 $\mu$m and about 300 $\mu$m, between about 10 $\mu$m and about 200 $\mu$m or between about 20 $\mu$m and about 100 $\mu$m such as about 50 $\mu$m.

6. The flow directed device of any one of the previous claims, further comprising at least one drag member (200) configured to be pushed by the fluid flow so to transport the device along a tubular structure.

7. The flow directed device of claim 6, wherein the ferromagnetic area or structure (300) is comprised within the drag member (200).

8. A system configured to release a flow directed device into a tubular structure comprising:

   a) the flow directed device of any one of the previous claims; and
   b) an insertion system (1000) comprising:

      an insertion member (1100) for accessing a tubular structure; and
      ii) a releasing unit (1200) operatively connected with the flow directed device and said insertion system (1000), the releasing unit (1200) being configured to gradually release the flow directed device without pushing it.

9. The system of claim 8, wherein the releasing unit (1200) comprises a spool around which the flow directed device is wound.

10. The system of claim 9, wherein the spool is configured as a removable cartridge.

11. The system of anyone of claims 8 to 10, further comprising a liquid reservoir (1400) operatively connected with said insertion member (1100) in a way to introduce a liquid into said insertion member (1100).

12. The system of claim 11, further comprising pumping means (1300) operatively connected with said a liquid reservoir (1400).

**Patentansprüche**

1. Strömungsgeführte Vorrichtung, dafür ausgelegt, in eine röhrenförmige Struktur eines Körpers eines Säugers, wie z.B. ein Blutgefäß, eingeführt zu werden, wobei die Vorrichtung ein proximales Ende (100), ein distales Ende (101) und einen langgestreckten Körper (102) dazwischen, der eine Längsachse (103) definiert, und eine(n) ferromagnetische(n) Bereich oder Struktur (300) an dem distalen Ende (101) angeordnet, der/die fähig ist, das distale Ende (101) auszulenken, wenn einem externen Magnetfeld ausgesetzt, umfasst,
   **dadurch gekennzeichnet, dass**
   wenigstens das distale Ende (101) als Band gestaltet ist.

2. Strömungsgeführte Vorrichtung nach Anspruch 1, wobei wenigstens das distale Ende (101) eine Biegesteifigkeit in dem Bereich zwischen etwa $10^{-10}$ und etwa $10^{-14}$ Pa·m$^{-4}$ aufweist.

3. Strömungsgeführte Vorrichtung nach einem der Ansprüche 1 oder 2, wobei wenigstens das distale Ende (101) und der langgestreckte Körper (102) einen Elastizitätsmodul in dem Bereich zwischen etwa 100 kPa und etwa 10 GPa, vorzugsweise zwischen 500 kPa und 5 GPa, aufweisen.

4. Strömungsgeführte Vorrichtung nach einem der vorstehenden Ansprüche, wobei der Querschnitt senkrecht zu der Längsachse (103) eine Abmessung in dem Bereich zwischen etwa 500 nm und etwa 30 $\mu$m, zwischen 1 und 10 $\mu$m, zwischen 2 und 20$\mu$m oder zwischen etwa 1 $\mu$m und etwa 5 $\mu$m aufweist und wobei die Breite in dem Bereich zwischen etwa 10 $\mu$m und etwa 500 $\mu$m, wie z.B. zwischen 30 und 300 $\mu$m, zwischen 50 und 200 $\mu$m oder zwischen 50 und 100 $\mu$m, liegt.

5. Strömungsgeführte Vorrichtung nach einem der Ansprüche 1 bis 3, wobei die Vorrichtung eine röhrenförmige Form mit Durchmessern in dem Bereich von etwa 500 nm bis etwa 500 $\mu$m, wie z.B. zwischen etwa 1 $\mu$m und etwa 300 $\mu$m, zwischen etwa 10 $\mu$m und etwa 200 $\mu$m oder zwischen etwa 20 $\mu$m und etwa 100 $\mu$m, wie z.B. 50 $\mu$m, aufweist.

6. Strömungsgeführte Vorrichtung nach einem der vorstehenden Ansprüche, ferner umfassend wenigstens ein Schleppelement (200), das dafür gestaltet ist, von dem Fluidstrom gedrückt zu werden, um die Vorrichtung entlang einer röhrenförmigen Struktur zu befördern.

7. Strömungsgeführte Vorrichtung nach Anspruch 6, wobei der/die ferromagnetische Bereich oder Struktur (300) in dem Schleppelement (200) enthalten ist.

**8.** System, dafür gestaltet, eine strömungsgeführte Vorrichtung in eine röhrenförmige Struktur freizugeben, umfassend:

> a) die strömungsgeführte Vorrichtung nach einem der vorstehenden Ansprüche; und
> b) ein Einführungssystem (1000) umfassend:
>
>> ein Einführelement (1100) zum Zugang zu einer röhrenförmigen Struktur; und
>> ii) eine Freigabeeinheit (1200), die mit der strömungsgeführten Vorrichtung und dem Einführsystem (1000) wirkverbunden ist, wobei die Freigabeeinheit (1200) zum allmählichen Freigeben der strömungsgeführten Vorrichtung ohne sie zu drücken gestaltet ist.

**9.** System nach Anspruch 8, wobei die Freigabeeinheit (1200) einen Spulenkörper umfasst, um die die strömungsgeführte Vorrichtung gewickelt ist.

**10.** System nach Anspruch 9, wobei der Spulenkörper als entfernbare Kartusche gestaltet ist.

**11.** System nach einem der Ansprüche 8 bis 10, ferner umfassend ein Flüssigkeitsreservoir (1400), das mit dem Einführelement (1100) auf eine Weise zum Einführen einer Flüssigkeit in das Einführelement (1100) wirkverbunden ist.

**12.** System nach Anspruch 11, ferner umfassend Pumpmittel (1300), die mit dem Flüssigkeitsreservoir (1400) wirkverbunden sind.

**Revendications**

**1.** Dispositif d'écoulement dirigé adapté pour être introduit dans une structure tubulaire d'un corps d'un mammifère, tel qu'un vaisseau sanguin, ledit dispositif comprenant une extrémité proximale (100), une extrémité distale (101) et un corps allongé (102) entre eux définissant un axe longitudinal (103), et une zone ou structure ferromagnétique (300) située sur ladite extrémité distale (101) capable de dévier l'extrémité distale (101) lorsqu'elle est soumise à un champ magnétique externe, **caractérisé en ce que**, au moins ladite extrémité distale (101) étant configurée sous la forme d'un ruban.

**2.** Dispositif d'écoulement dirigé selon la revendication 1, au moins l'extrémité =: distale (101) ayant une rigidité à la flexion comprise entre environ $10^{-10}$ et environ $10^{-14}$ Pa·m⁻4.

**3.** Dispositif d'écoulement dirigé selon l'une quelconque des revendications 1 ou 2, au moins l'extrémité distale (101) et le corps allongé (102) ayant un module élastique compris entre environ 100 kPa et environ 10 GPa, de préférence entre 500 kPa et 5 GPa.

**4.** Dispositif d'écoulement dirigé selon l'une quelconque des revendications précédentes, la section transversale perpendiculaire à l'axe longitudinal (103) ayant une dimension comprise entre environ 500 nm et environ 30 $\mu$m, entre 1 et 10 $\mu$m, entre 2 et 20 $\mu$m ou entre environ 1 $\mu$m et environ 5 $\mu$m et la largeur étant comprise entre environ 10 $\mu$m et environ 500 $\mu$m, par exemple entre 30 et 300 $\mu$m, entre 50 et 200 $\mu$m ou entre 50 et 100 $\mu$m.

**5.** Dispositif d'écoulement dirigé selon l'une quelconque des revendications 1 à 3, le dispositif ayant une forme tubulaire avec des diamètres compris entre environ 500 nm et environ 500 $\mu$m, par exemple entre environ 1 $\mu$m et environ 300 $\mu$m, entre environ 10 $\mu$m et environ 200 $\mu$m ou entre environ 20 $\mu$m et environ 100 $\mu$m, par exemple environ 50 $\mu$m.

**6.** Dispositif d'écoulement dirigé selon l'une quelconque des revendications précédentes, comprenant en outre au moins un élément de traînée (200) configuré pour être poussé par l'écoulement de fluide afin de transporter le dispositif le long d'une structure tubulaire.

**7.** Dispositif d'écoulement dirigé selon la revendication 6, la zone ou structure ferromagnétique (300) étant comprise à l'intérieur de l'élément de traînée (200).

**8.** Système configuré pour relâcher un dispositif d'écoulement dirigé par un flux dans une structure tubulaire comprenant :

> a) le dispositif d'écoulement dirigé selon l'une quelconque des revendications précédentes ; et
> b) un système d'insertion (1000) comprenant :
>
>> un élément d'insertion (1100) permettant d'accéder à une structure tubulaire ; et
>> ii) une unité de libération (1200) reliée fonctionnellement au dispositif d'écoulement dirigé et audit système d'insertion (1000), l'unité de libération (1200) étant configurée pour libérer progressivement le dispositif d'écoulement dirigé sans le pousser.

**9.** Système selon la revendication 8, l'unité de libération (1200) comprenant une bobine autour de laquelle est enroulé le dispositif d'écoulement dirigé.

**10.** Système selon la revendication 9, la bobine étant configurée comme une cartouche amovible.

11. Système selon l'une quelconque des revendications 8 à 10, comprenant en outre un réservoir de liquide (1400) relié fonctionnellement audit élément d'insertion (1100) de manière à introduire un liquide dans ledit élément d'insertion (1100).

12. Système selon la revendication 11, comprenant en outre un moyen de pompage (1300) relié fonctionnellement audit réservoir de liquide (1400).

Figure 1

Figure 2

Figure 3

Figure 4

Figure 5

Figure 6

Figure 7

Figure 8

Figure 9

Figure 10

Figure 11

Figure 12

35

Figure 13

Figure 14

Figure 15

Figure 15 (continued)

Figure 16

Figure 17

Figure 18

Figure 19

Figure 20

Figure 21

Figure 22

Figure 23

Figure 24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6524303 B
- US 20070270781 A
- WO 2004095536 A
- WO 2016110564 A
- WO 2018100005 A1

**Non-patent literature cited in the description**

- **ROSSET** ; **SHEA**. *Applied Physics A*, February 2013, vol. 110 (2), 281-307